# EUROPEAN PATENT APPLICATION

(11) **EP 2 098 534 A1**
(43) Date of publication of application: **09.09.2009**
(21) Application number: 09003184.0
(22) Date of filing: 05.03.2009
(51) Int. Cl.: C07H 15/252, A61K 31/704, A61P 35/00

(54) **Esters of glucuronide prodrugs of anthracyclines and method of preparation and use in tumor-selective chemotherapy**

(30) Priority: 07.03.2008 EP 08004249
(71) Applicant: PHARMACHEMIE B.V., 2031 GA Haarlem (NL)
(72) Inventor: Aben, Rene Wilhelmus Marie, 6525 SM Nijmegen (NL); Scheeren, Johan Wilhelm, 6581 CL Malden (NL); Cornelissen, Jeroen Johannes Lambertus Maria, 5473 EL Dinther (NL); De Vos, Dick, 2341 LP Oegstgeest (NL); Haisma, Hidde Jacob, 3871 JE Hoevelaken (NL)
(74) Representative: Griebling, Onno

(57) **Abstract**

The invention relates to novel esters and in particular to some novel esters of glucuronide prodrugs of anthracyclines having tunable water-solubility, their synthesis and use in tumor-selective chemotherapy. It appeared that in the final step in the synthesis of these prodrugs, i.e. the coupling of the glucuronide spacer moiety to the parent drug molecule, protection of the sugar hydroxyls is, surprisingly, no longer required. A process for the preparation of these unprotected sugar spacer moieties is also disclosed.

## Description

This invention relates to novel esters and in particular to some novel water-soluble esters of glucuronide prodrugs of anthracyclines, their synthesis and use in tumor-selective chemotherapy.

More specifically this invention relates to anthracycline derivatives **1** 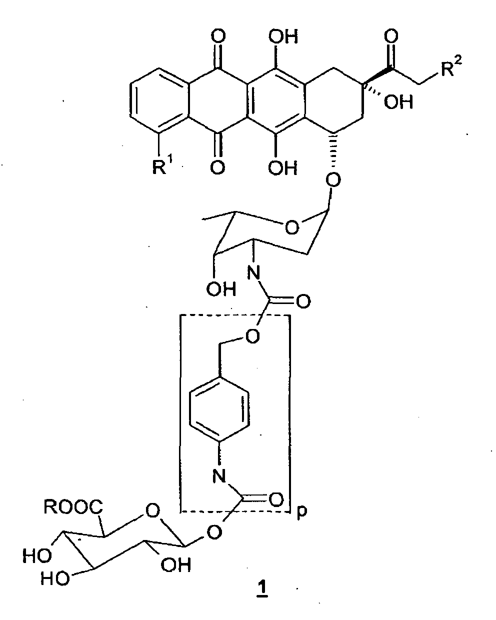 wherein R, R¹, R², n, p, x and alkyl are as defined in claim 1.

It has now surprisingly been found that these esters can be prepared without the necessity of any protection of the sugar hydroxyl groups, in any step of the preparation process.

This was totally unexpected, because it was always deemed necessary by the skilled persons that the sugar hydroxyl groups should be protected during the synthesis steps, to obviate side-reactions.

The invention also relates to a process for the preparation of some specific anthracycline prodrug esters, which includes the coupling of an anthracycline to a glucuronide spacer moiety having unprotected sugar hydroxyl groups and an activated benzylic hydroxyl group. Further, the spacer moiety is not restricted to a single moiety, but may consist of a combination of several moieties, as needed, and as known to the expert.

The inventors further found that the water solubility of the present esters can be "tuned" when the prodrugs have, for example, mono-methoxy-polyethylene glycol groups with different chain lengths. "Tuning" is, in this respect, meant to indicate that the water-solubility can be changed from less water-soluble to good water-soluble, by a proper selection of the chain length of the polyethylene glycol groups being present in the ester part of the unprotected sugar moiety. A very efficient preparation process for such "tunable" esters, providing the compounds in a high yield, is also disclosed. The invention further relates, in another aspect, to the preparation of said glucuronide spacer moiety having unprotected sugar hydroxyl groups and an activated benzylic hydroxyl group.

The invention further relates, in another aspect, to aminocamthotecin prodrug esters, as defined below, being coupled to said glucuronide spacer moiety having unprotected sugar hydroxyl groups and an activated benzylic hydroxyl group.

The invention relates, more specifically, to a process for the preparation of said esters of glucuronide prodrugs of anthracyclines, having formula 1 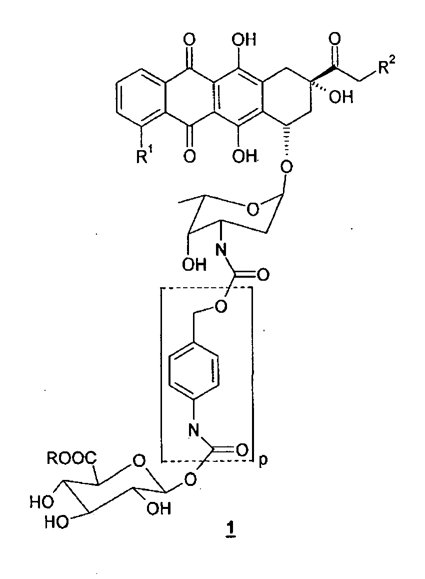 wherein:
- R¹ =: H or OCH₃;
- R² =: H or OH;
- R =: CH₃; CH₂=CH-CH₂-; or CH≡C- (CH₂)ₓ-;
comprising the reaction of a compound having formula 10a, 10b or 10c, having unprotected sugar hydroxyl groups, 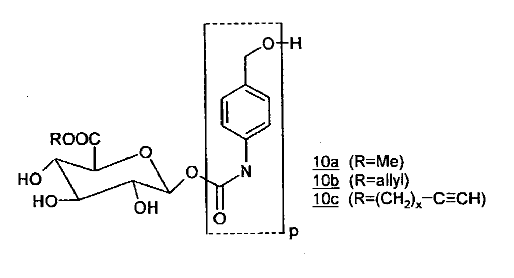 wherein p is an integer from 1 to 5, preferably 1 to 3, more preferably 1 or 2, with p-nitrophenylchloroformate, to obtain a compound having formula 11a, 11b or 11c respectively; 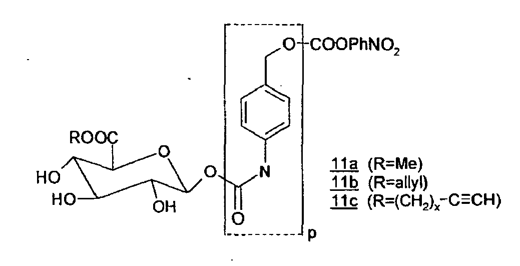 followed by the reaction with an anthracycline of formula 21 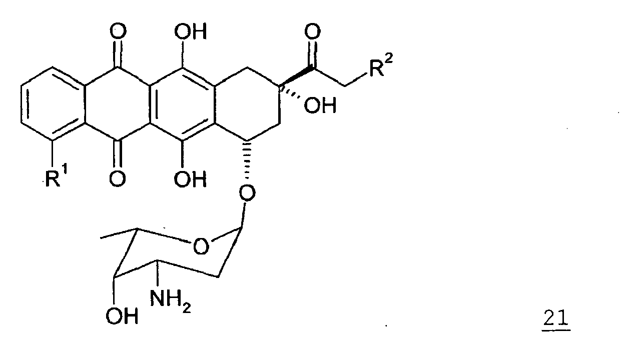 wherein R₁ and R₂ are as defined above, to obtain an anthracycline prodrug ester having a formula 1 wherein R is CH₃, allyl or (CH₂)ₓ-C≡CH. The prodrugs wherein R = (CH₂)ₓC≡CH can be used for the preparation of certain water-soluble esters by reaction with a methoxy polyethylene glycol azide to obtain the corresponding monomethoxy polyethylene glycol triazyl esters, whereof the water-solubility can be tuned, as desired.

The first key step in the preparation of the present prodrugs is the preparation of a compound of formula 10a or 10b, by reaction of an isocyanate of formula 3: 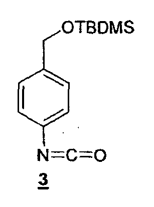 wherein TBDMS represents the t-butyldimethylsilyl protective group, with a completely unprotected glucuronic acid ester of formula 9a or 9b, respectively, 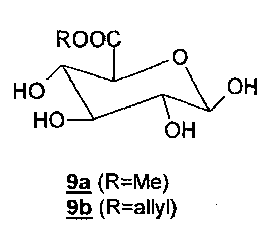 to obtain a compound of formula 13a or 13b, respectively, 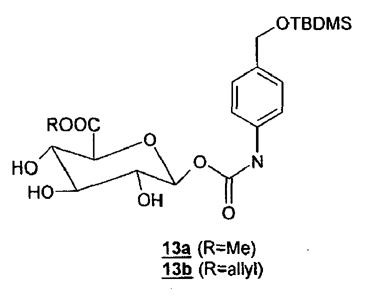 followed by deprotection of the silyl protective group to obtain a compound of formula 10a or 10b respectively.

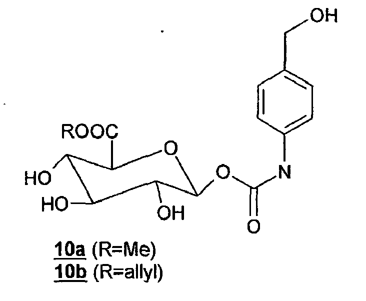

The second key step in the preparation of the present prodrugs is the preparation of a compound having formula 10c, 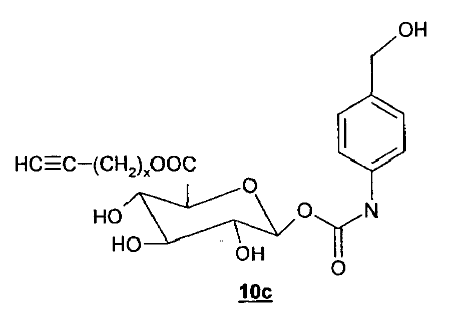 by reaction of a compound of formula 13a or 13b 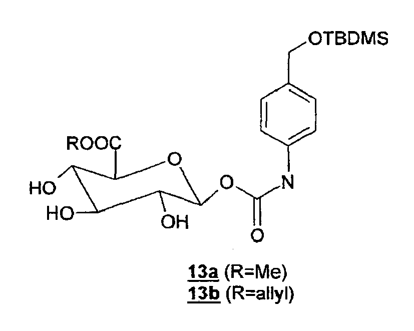 with an alcohol of formula CH≡-C-(CH₂)ₓ-OH, wherein x is an integer, preferably from 1-5, in the presence of a base, followed by acidic removal of the silyl protective group, to obtain a compound of formula 10c.

Further, a process will be given for the preparation of anthracycline derivatives having formula 1, as mentioned above, wherein R is a residue having formula 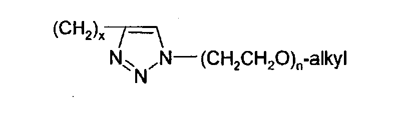 wherein x, n, and alkyl are as defined in claim 1, by reaction of a compound of formula 1, wherein R is CH≡C-(CH₂)ₓ-, with N₃-(CH₂CH₂O)ₙ-alkyl in the presence of a copper catalyst to obtain the corresponding mono-alkoxy-polyetheneneglycol triazol ester of formula 1.

An example of an anthracycline is doxorubicin, having the following formula 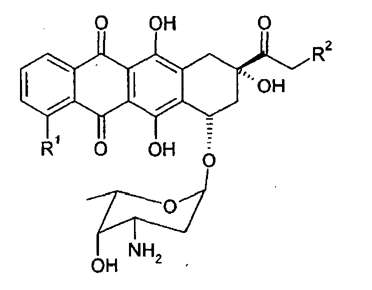 wherein R¹ = OMe, and R² = OH; hereafter indicated as DOX. Some derivatives of DOX, to be discussed below, are: 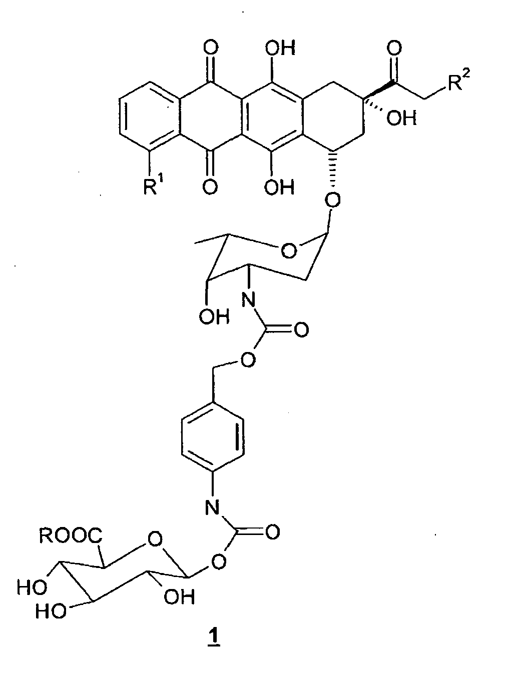
- DOX-GA3: (R¹ = OMe, R² = OH, R = Na, H)
- DOX-mGA3: (R¹ = OMe, R² = OH, R = Me)
- DOX-alGA3: (R¹ = OMe, R² = OH, R = allyl)
- DOX-proparGA3: (R¹ = OMe, R² = OH, R = propargyl)
- DOX-mPEGn-GA3: (R¹ = OMe, R² = OH, R is

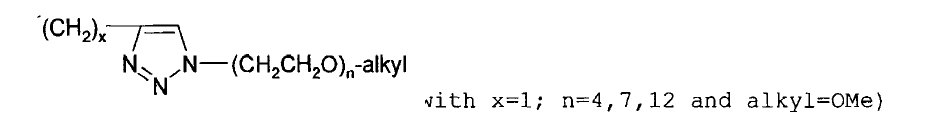

Anthracyclines represent a class of anticancer agents that have been widely used for the development of prodrugs (review: F. Kratz et al, Curr. Med. Chem. 2006, 13, 477-523). Glucuronide prodrugs of anthracyclines such as DOX-GA3 (formula 1) can be selectively activated in tumors by extracellular human β-glucuronidase resulting in a better therapeutic-index than the parent drug doxorubicin (review: M. de Graaf et al, Curr Pharm Des 2002, 8, 1391-1403). The maximum tolerated dose (MTD) of DOX-GA3 in tumor bearing mice based on 10% weight loss was approximately 500 mg/kg i.v., compared with 8 mg/kg for doxorubicine (P.H. Houba et al, Br J Cancer 2000, 84, 1-8). Administration of half of the MTD to tumor bearing mice has resulted in significantly lower concentrations of doxorubine in plasma whereas the concentrations in tumor tissue were higher than after administration of doxorubine (P.H. Houba et al, Br J Cancer 2000, 84, 1-8). A disadvantage of hydrophilic prodrugs such as DOX-GA3 is their rapid elimination by the kidneys. In earlier experiments in tumor bearing nude mice, it was shown that DOX-GA3 had completely disappeared from plasma within four hours after administration of 250mg/kg of the prodrug. This poses a major problem for the use in cancer treatment, as this means that very high doses are required (P.H. Houba et al, Br J Cancer 2000, 84, 1-8). Recently a methyl ester of DOX-GA3, from now on indicated as DOX-mGA3, was investigated. It was hypothesized, that a methyl ester of DOX-GA3 would be more lipophilic and therefore of better use in enzyme prodrug therapy. Methyl esters of other methylated glucuronide prodrugs have been reported to be hydrolyzed by carboxyl esterases. It was expected that in this way slow release of DOX-GA3 after administration of DOX-mGA3 might result. Indeed DOX-mGA3 was enzymatically converted to DOX-GA3 but with a half time of approximately 05 min in mouse plasma to 2.5h in human plasma. Despite its fast conversion to DOX-GA3 in mouse plasma DOX-mGA3 showed improved pharmacokinetics in mice (M. de Graaf et al, Biochem Pharmacol 2004, 68, 2273-2281). A disadvantage however remains the very bad solubility in water so that it has to be administered in a vehicle containing Cremophore EL which is considered to cause some hypersensitive reactions (A. Sharma et al, Cancer Letters 1996, 107, 265-272).

It has now been found that the abovementioned disadvantages can be obviated by a specific group of glucuronide prodrugs of anthracyclines. For, the inventors found a new route for preparing propargyl esters of, for example DOX-GA3 which can be conjugated with mono-methoxy-polyethylene glycol groups to give water soluble DOX-GA3 esters. By introducing monomethoxy-polyethylene glycol groups with different chain lengths solubility can be tuned. Furthermore these esters are more slowly hydrolyzed by enzymes.

The invention will be explained hereafter by means of the anthracycline doxorubicin (DOX), as an example of a drug having an active amino group, but the invention is not restricted to anthracyclines, but is also applicable for other drugs having an active amino group, such as for example aminocampthotecin, or more generally to compounds which can be coupled to the benzylic hydroxyl group of the glucuronide spacer moiety having unprotected sugar hydroxyl groups, as will be explained below.

Therefore, said corresponding aminocampthotecin prodrugs have a comparable solubility and hydrolysis profile as the anthracycline prodrugs, due to the same leaving groups coupled to the parent drug molecule.

The value of p is, for the sake of simplicity, in the explanation of the following reaction schemes, in most compounds, equal to 1, but it will be obvious that the invention should not be restricted to compounds wherein p = 1.

### Experimental section.

A priory. Anthracycline-GA3 esters could be prepared analogously to the preparation of DOX-mGA3 as outlined in scheme 1 but now using a glucuronic acid ester precursor having the appropriate ester group (M. de Graaf et al, Biochem Pharmacol 2004, 68, 2273-2281). It proved very difficult however to remove selectively the acetate groups in the last step. This is best achieved with base in methanol which causes however also exchange of the glucuronic acid ester group by a methoxy group. A variety of other methods including the use of base in the alcohol corresponding with the ester group gave low yields and much side reactions.

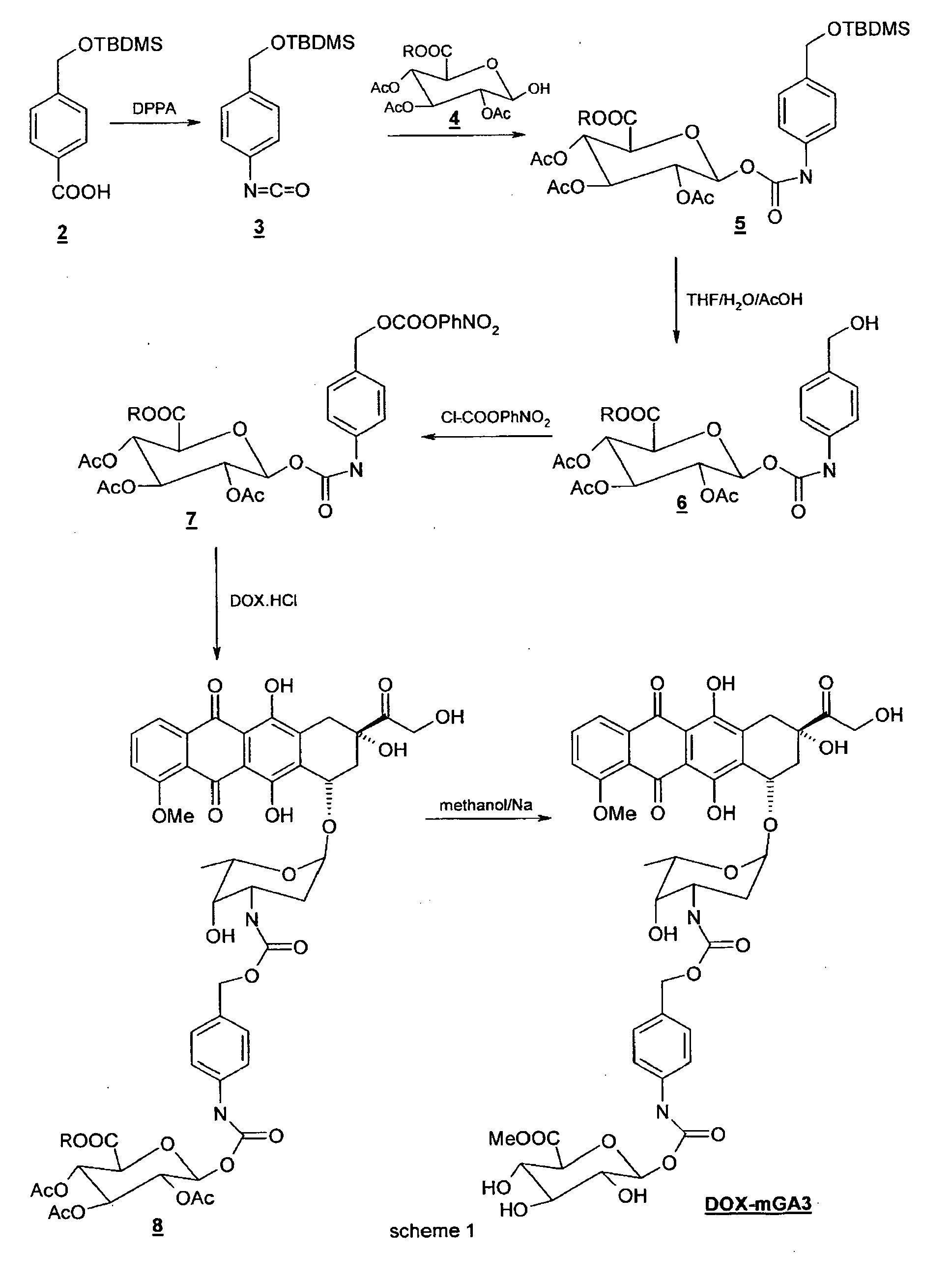

We found however that transesterification was possible from DOX-mGA3 with a variety of alcohols by treating DOX-mGA3 with a large excess of the alcohol in the presence of a base (scheme 2). After conversion and neutralization with silica the excess of alcohol has to be removed by evaporation in vacuo or by extraction with ether. Disappointingly it appeared difficult to obtain the isolated esters free from the starting methyl ester.

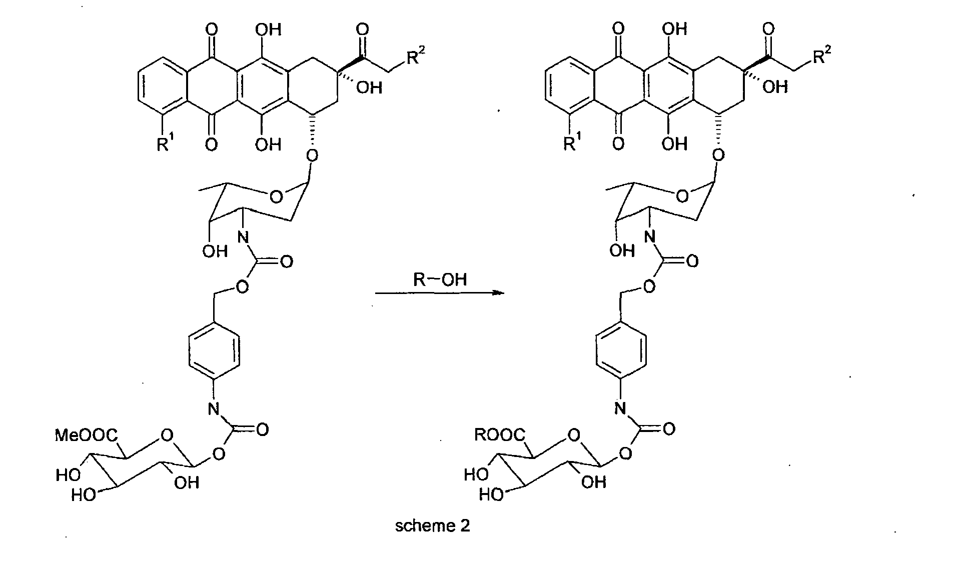

The method becomes even less efficient for larger alcohols such as mono-methoxy-polyethylene glycol. Yields were so low and purification proved so difficult that we searched for a new and general method for the introduction of polyethylene glycol ester groups which could circumvent all this problems. We, then found that the polyethylene glycol groups can be introduced via a click reaction, (which procedure is as such known from H.C. Kolb et al. Drugs Discovery Today 2003, 8, 1128-1137; J.A. Opsteen et al Chem. Comm. 2005, 57-59)starting with a propargyl or longer chain terminal alkyn ester as outlined in scheme 3 for propargyl esters.

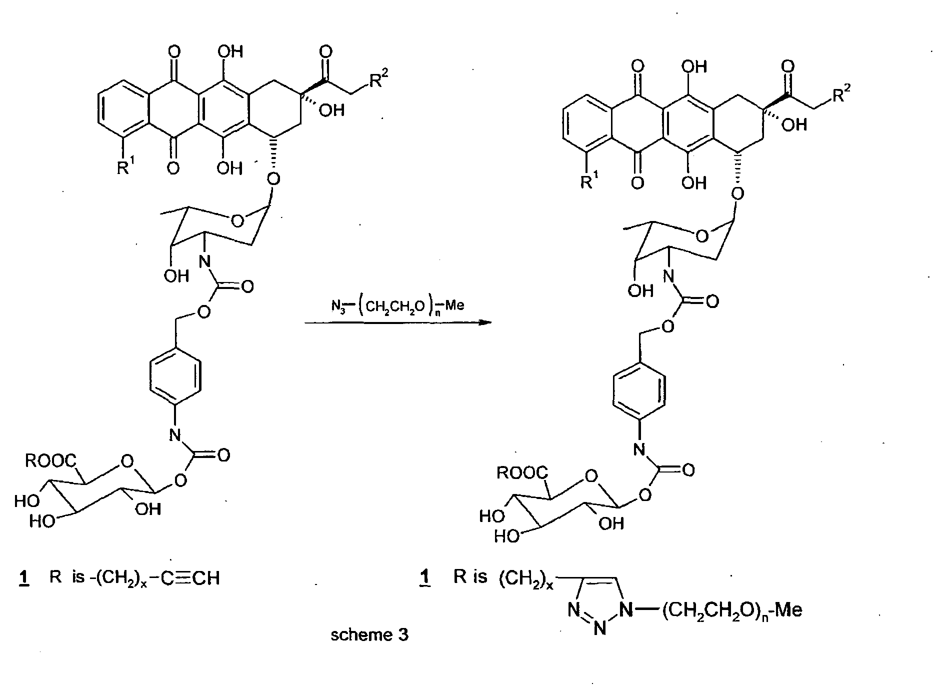

Indeed by treating propargyl esters **1** (R=propargyl)) with the mono-methoxy-polyethylene glycol azides in the presence of a copper catalyst the corresponding mono-methoxy-polyethylene glycol triazyl esters **1** (x=1) were obtained in moderate to good yields.

These results stimulated us to search for a more efficient synthesis of the anthracycline alkynyl esters **1** (R is (CH₂)ₓC≡CH) as the exchange reaction described in scheme 2 never gave yields above 40% and also did not lead to pure products free from methyl esters **1**.

To obtain the synthesis improvement, we took the allyl esters as the starting compounds for this exchange reaction instead of the methyl esters. Usually, the allyloxy group is a better leaving group than the methoxy group. Further, allyl glucuronate, now used as a starting material, is more easy and in better yields available than methyl glucuronate.

Finally a synthesis had to be found in which removal of the sugar protecting groups, usually acetate groups, in the last step is no longer necessary.

To our surprise we found that protection of the sugar hydroxyl groups in the synthesis of the DOX-GA3 allyl ester or DOX-mGA3 ester is in fact not necessary, as described in scheme 4.

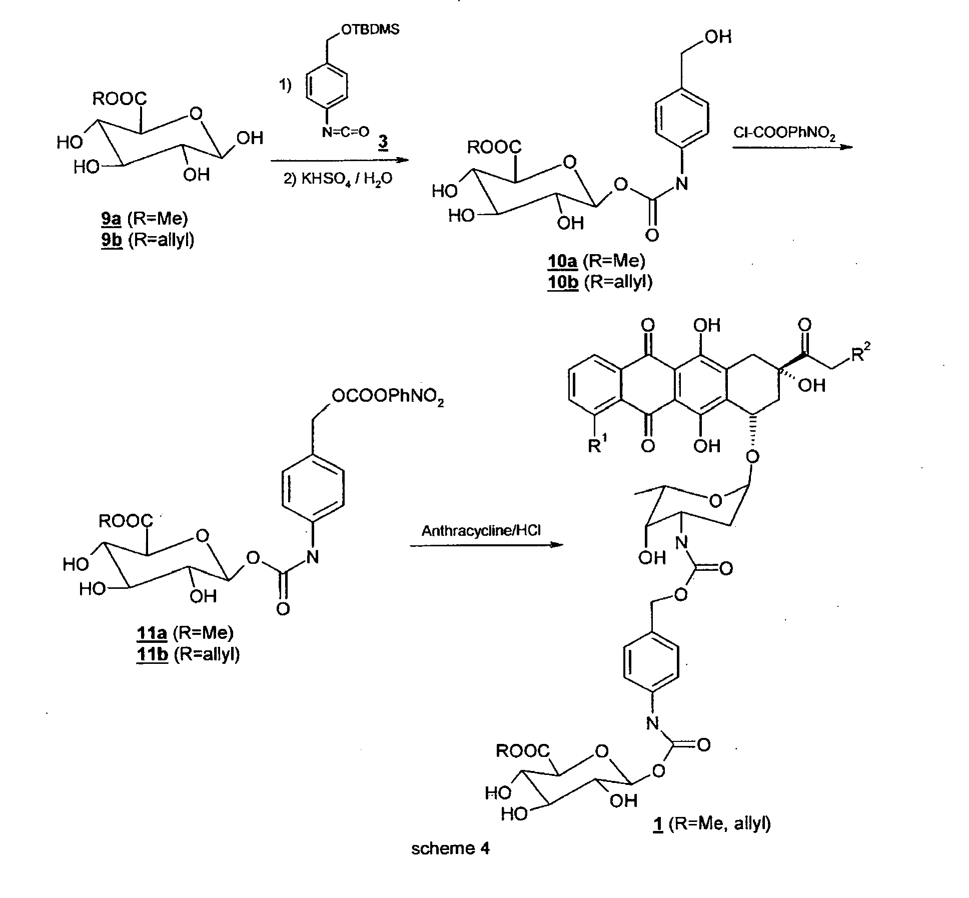

The present inventors found, surprisingly, that the anomeric hydroxyl groups, and to a lesser extent also the benzylic hydroxyl groups, appeared to be sufficiently more reactive than the secondary sugar hydroxyl groups, so that protection of the sugar hydroxyl groups can be avoided. Most important is however that in the final conversion of the glucuronide spacer moiety (for example conversion of **11** to **1**) protection of the sugar hydroxyls is no longer necessary. Unfortunately propargyl glucuronate cannot be prepared in the same way as allyl glucuronate from glucuronic acid (A. Abdessamama Elet al, J. Org. Chem. 2006, 71, 9628-36) or methyl glucuronate from d(+)-glucurone-3,6-lactone (Bollenback et al; J. Am. Chem. Soc. 1955, 77, 3310-15).

We solved this problem by trans esterification with propargyl alcohol from the methyl or allyl esters **5a, b** and **13a, b** as outlined in scheme 5 and 6.

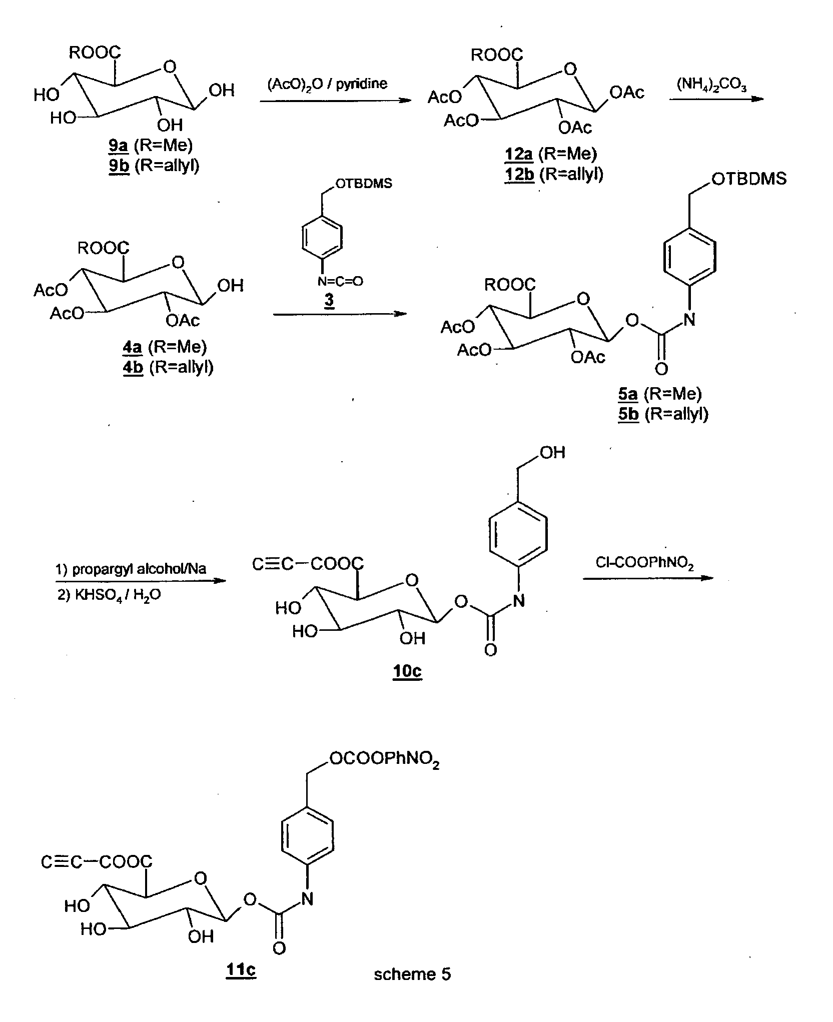

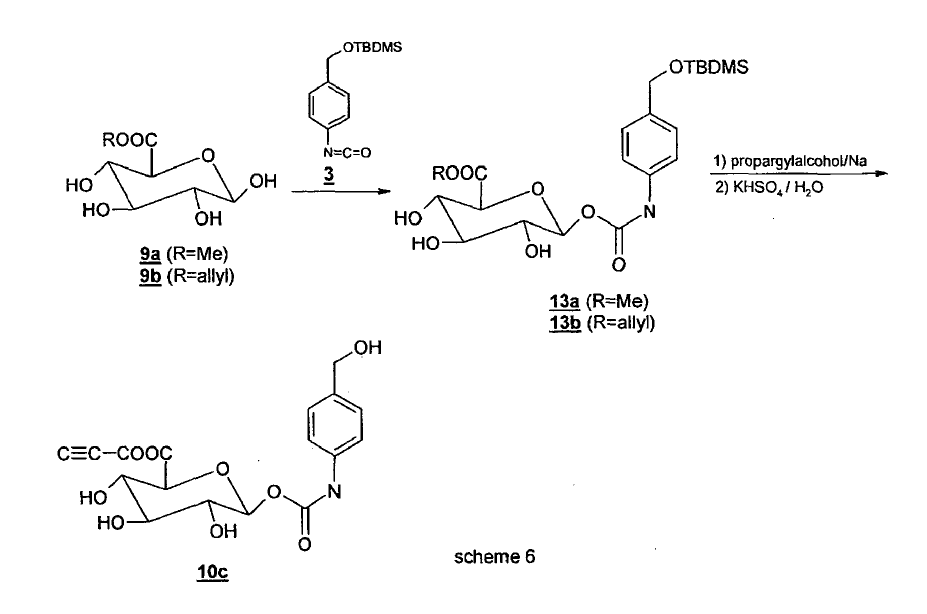

Although the reaction sequence of scheme 5 has more steps than the sequence of scheme 6 the overall yield following scheme 5 was much higher, probably due to the presence of water in compound **13a, b**. This leads to partial hydrolysis of the ester group during the basic trans esterification. More efficient removal of water from **13a, b** may therefore increase the yields according scheme 6.

Compound **11c** was coupled in good yields with anthracyclines in the same way as described in scheme 4 for the methyl (**11a**) or the allyl derivatives **(11b).**

The findings described in this patent have a strong impact on a variety of related reactions in this field. Up to now the general procedure was that compounds like **13** have to be protected at the sugar part before the TBDMS group is selectively removed and the benzylic OH is activated for a coupling reaction. In a lot of related cases of coupling with anomeric sugar hydroxyls protection of the other sugar hydroxyls may be no longer necessary. We found that also for the synthesis of prodrugs with a double spacer protection of the sugar hydroxyls before coupling of the double spacer-sugar moiety with an anthracycline is not necessary as illustrated in scheme 8 for the synthesis of the prodrugs **1** having a double spacer moiety.

The synthesis of the double spacer-sugar moieties **15a**, **b, c** are described in scheme 7.

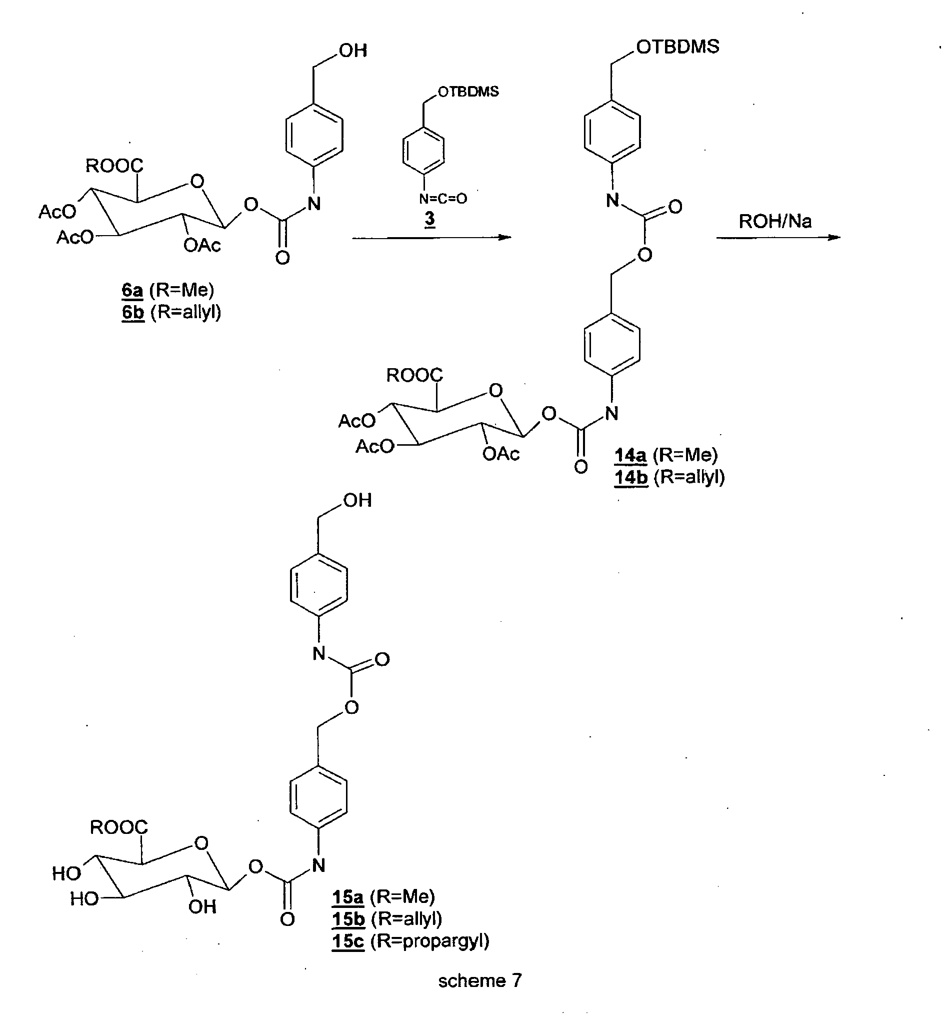

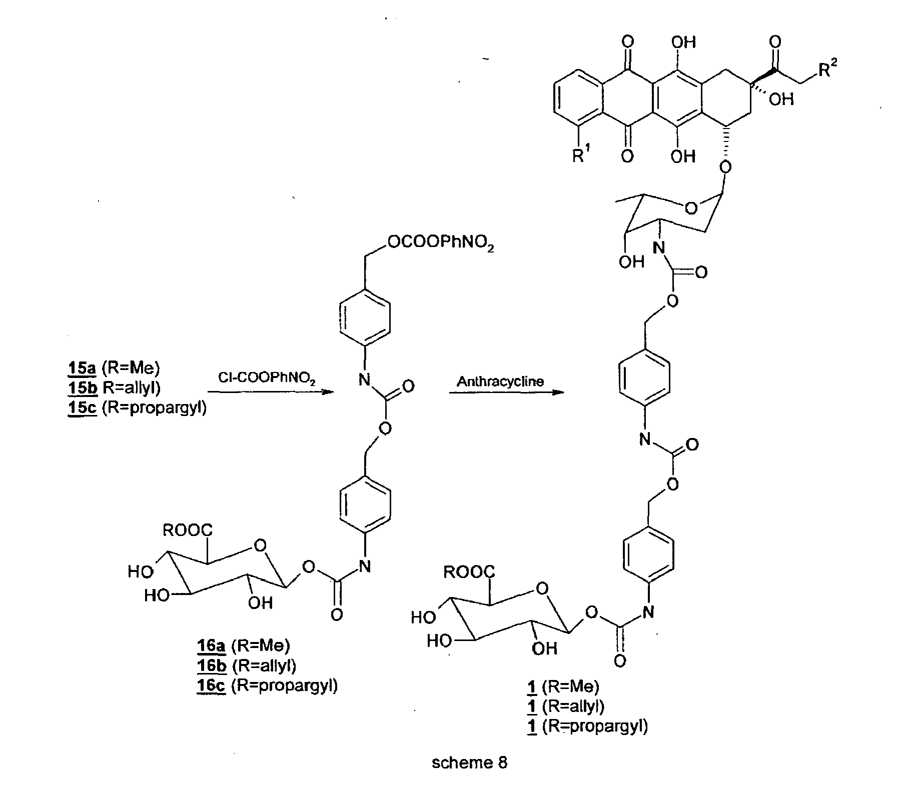

Our results with click chemistry as presented in scheme 3 can also be applied to **1** (R is (CH₂)ₓC≡CH) in click reactions with azides of branched polyethylene glycols or even dendrimers containing azide functionalities (see for example E. Fernandez-Megia et al Biomacromolecules 2006, 7, 3104-3111) or compounds **17** and **18**, which could be easily available from the corresponding tosylates ( Jian-Sen Li et al European J. Org. Chem. 2000, 485-490).

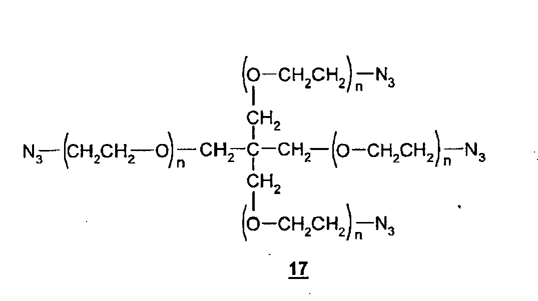

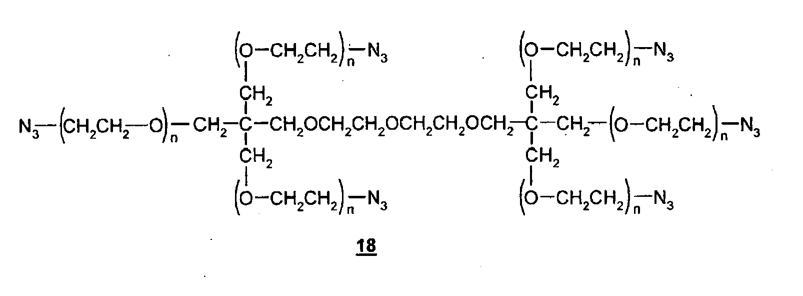

Glucuronic esters connected to a benzylic spacer e.g. compounds **11** and **16** can also be coupled with amino group containing drugs other than anthracycline such as 9-aminocampthotecin **(19)** resulting into compounds **20a** and **20b** respectively.

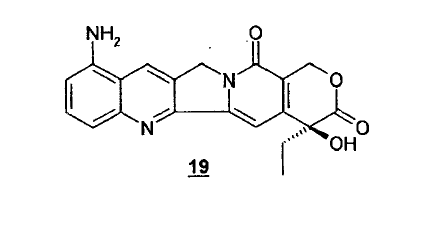

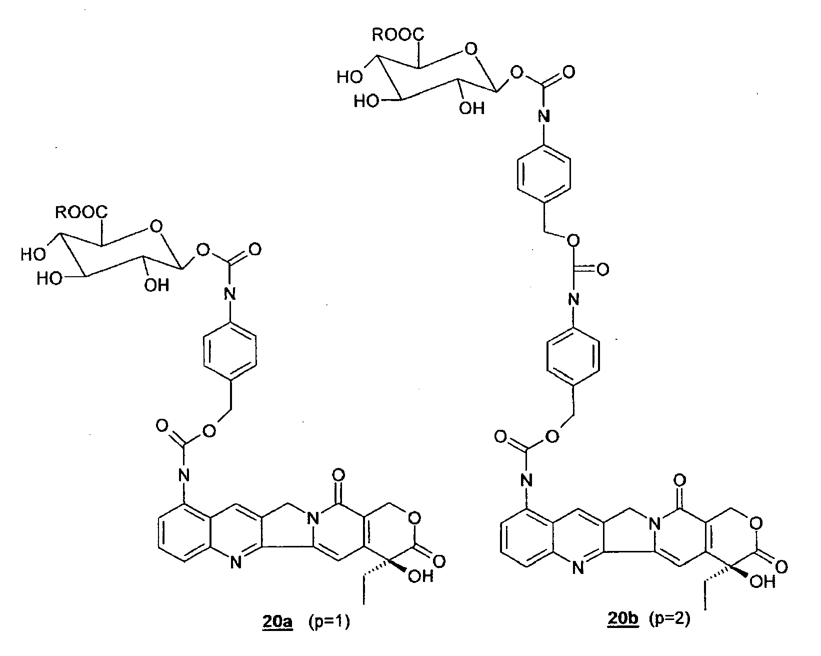

### Hydrolysis of prodrug esters 1 in buffer and in plasma (scheme 9).

The esters described in this patent are in fact precursors of glucuronide prodrugs which are activated by β-glucuronidase.

Due to their higher (and tunable) lipophilicity they may cause a better distribution of the corresponding glucuronide prodrug in tumor tissue as was already demonstrated for the ester DOX-mGA3 (M. de Graaf et al, Biochem Pharmacol 2004, 68, 2273-2281).

It can be expected that prodrug esters **1** with R is 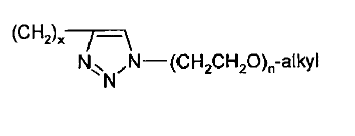 hydrolyze faster than alkyl esters like DOX-mGA3 due to the better leaving ability of the ester group of **1**. This is caused by the electron-withdrawing properties of the triazol functionality. Indeed in a phosphate buffer with pH=7.33 it took 24 hrs at room temperature before DOX-mGA3 was completely hydrolyzed. The DOX derivatives of **1** with n=7 or 12 and x=1( DOX-mPEG7GA3 and DOX-mPEG12GA3) were, nevertheless, already hydrolyzed within 4 hrs under these circumstances.

The effect of the electron-withdrawing ability of triazol group will be diminished by prolonging the alkyl tail bearing the triazol group so that the electron-withdrawing effect of this group is weakened when x>1. In this way, the water-solubility of the present esters can be modified (tuned), as desired, on the basis of the requested (or desired) solubility profile of the prodrug.

These triazols can be prepared from the corresponding esters **1** (R is 3-butynyl and 4-pentynyl respectively).

Experiments showed that addition of esterases had only weak effects on the hydrolysis of DOX-triazol ester **1**.

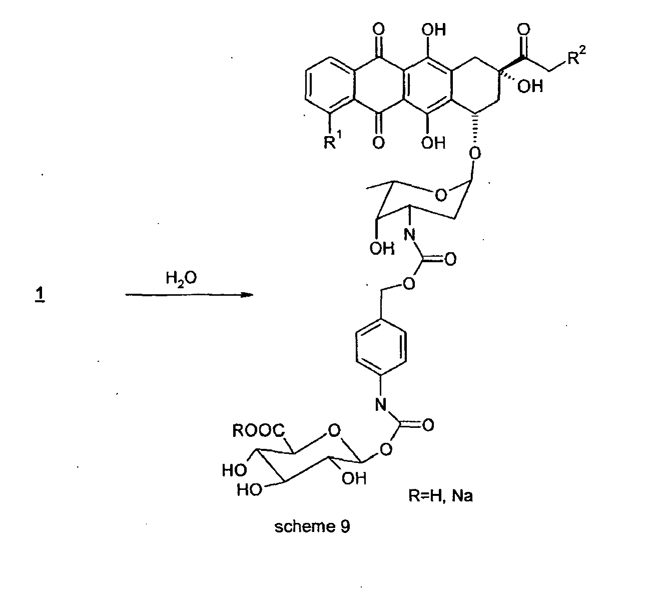

### Solubility in water of 1

Whereas DOX-mGA3 is completely insoluble in water, the corresponding DOX-triazol esters **1** dissolve increasingly well with increasing n. For DOX-triazol ester **1** (DOX-mPEG4GA3) about 0.6 mg prodrug dissolve in 1ml of water whereas for DOX-mPEG7GA3 already 2.5 mg dissolve in 1 ml of water and for DOX-mPEG12GA3 this has reached more than 15mg in 1 ml of water.

### In vitro antiproliferative effects in OVCAR-3 cells

The toxicity of the prodrugs was tested with OVCAR-3 cells. The cells were incubated for 3 days with the different prodrugs DOX-alGA3, DOX-mPEG4-GA3, DOX-mPEG7-GA3, and for comparison also with Doxorubicin, in fetal bovine serum (FBS), in the presence or absence of bovine beta-glucuronidase (GUS). The concentrations used for the prodrugs ranged from 0.001 µM to 50 µM.

The IC50 values are represented in the following table:
IC50 (M)

| | No GUS | with GUS (10 units/µL) |
|---|---|---|
| DOX | 6.19E-08 | |
| DOX alGA3 | 9.81E-06 | 5.84E-08 |
| DOX mPEG4-GA3 | 2.21E-05 | 6.21E-08 |
| DOX mPEG7-GA3 | 8.33E-06 | 7.74E-08 |

The results from the table demonstrate that without GUS, the prodrugs are between 150 and 300 times less toxic than doxorubicin. In the presence of GUS, the prodrugs DOX-alGA3 and DOX-mPEG4-GA3 have a comparable toxicity as doxorubicin, and DOX-mPEG7-GA3 is slightly less toxic.

**This invention is further explained in the following examples**

**General remarks:** H¹-NMR were recorded on a Bruker AC-300. NMR spectra were recorded at 298K using solvents as indicated. Chemical shifts were recorded in parts per million downfield relative to tetramethylsilane. Coupling constants (J) are given in Hertz (Hz). Mass spectra were recorded on aVG7070E double focusing mass spectrometer.

For the assignment of NMR peaks of the anthracycline prodrugs the numbering in the figure below has been used.

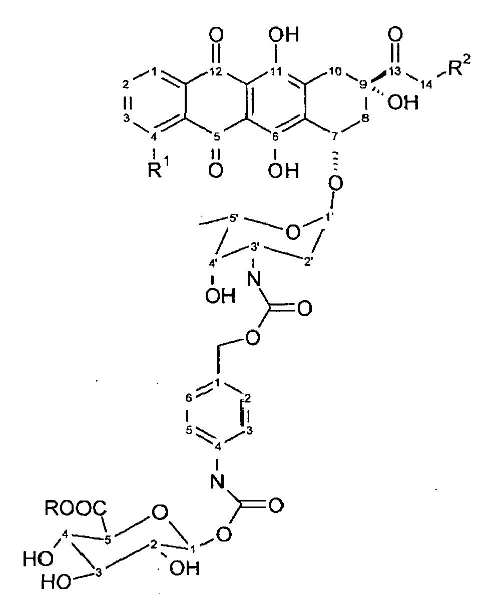

### Example 1.

### Conversion of DOX-mGA3 with propargyl alcohol into DOX-propGA3 1 (R¹=Me, R²=OH, R= propargyl).

In a flame dried reaction vessel was dissolved (100mg, 0,108 mmol) DOX-mGA3 in 5ml of dry THF and 30ml of propargyl alcohol, distilled from potassium carbonate under reduced pressure. To the mixture was added 1.5 ml of a solution prepared from 12 mg of Na and 5 ml of propargyl alcohol. The mixture was kept for 1.5h at room temperature and then neutralized by adding 2g of silica. After removal of the solvents in vacuo at room temperature the remaining silica in which the reaction compounds were absorbed is added to a silica column. Chromatography (eluens 10% methanol/dichloromethane followed by 15% methanol/dichloromethane). Yield: 70mg (58%) of DOX-propGA3 **1** (R= propargyl). NMR showed that the obtained propargyl ester still contains about 15% of the starting compound DOX-mGA3.
(M⁺Na)_{calc}= 973.24907
(M+Na)_{fnd} = 973.2490

In the same way DOX-GA3 esters were prepared with R=CH₂CH₂OMe Yield: 53% (with about 15% of the starting compound)
(M+Na)_{cal}=993.27529
(M+Na)_{fnd=}993.28255

And with R³=CH₂CH₂OH
With LiO-C-C-OH as base.
Yield: 50% (with about 10% of the starting compound)
(M+Na)_{cal}=979.25964
(M+Na)_{fnd}=979.26535

### Example 2.

### Synthesis of methyl 6-([4-([1-(tert-butyl)-1,1-dimethylsilyl]oxymethyl) anilino] carbonyloxy)-3,4,5-trihydroxytetrahydro-2H-2-pyrancarboxylate 13a from methyl 3,4,5,6-tetrahydroxy- tetrahydro-2H-2-pyrancarboxylate 9a. (scheme 6)

In a flame dried reaction vessel first *4-([1-(tert-butyl)-1,1-dimethylsilyl]oxymethyl)phenyl isocyanate* **3** was prepared by dissolving 2 g (7.52 mmol) of HOOC-Ph-CH₂OTBDMS in 40 ml of dry toluene and adding 0.9 g (8.9 mmol) of Et₃N and 2.45 g (8.9 mmol) of (DPPA) diphenylphosphoryl azide. The mixture was kept overnight under an argon atmosphere and after that heated in an oil bath at 85° for 2.5 h.

After the reaction mixture had cooled down to room temperature 1.32 g of **9a** (4.8 mmol) dissolved in 15 ml of acetonitrile was added and the reaction mixture was left overnight at room temperature. After evaporation of the solvent in vacuo (35°/0.8mm) the residue was purified by column chromatography (10% methanol/dichloromethane).
Yield: 0.85 g (38%) of **13a.**
Mass: 494 (M+Na); 965 (2M+Na)
H¹NMR: CD₃OD; δ 0.09 (s, 6H, Si-Me₂), 0.93 (s, 9H, Si-tBut), 3.38-3.60 (m, 3H, Glu 2,3,4-H), 3.76 (s, 3H, COOCH₃), 3.96 (d, J=9.2Hz, Glu 5-H), 4.68 (s, 2H, ArCH₂), 4.83 (s, Glu 2,3,4-OH), 5.48 (d, 1H, J=5.9Hz, Glu 1-H), 7.24 (d, 2H, J=8.2Hz, Ar), 7.40 (d, 2H, J=8.2Hz, Ar)

In the same way *allyl 6-([4-([1-(tert-butyl)-1,1-dimethylsilyl]oxymethyl)anilino]carbonyloxy) -3,4;5-trihydroxytetrahydro-2H-2-pyrancarboxylate* **13b** was prepared from *allyl 3,4,5,6-tetrahydroxytetrahydro-2H-2-pyrancarboxylate* **9b**.
Purification by flash chromatography
(hepthane/ethylacetate/methanol=70/30/2).
Yield: 40% of **13b.**
(M+Na)_{ca}l=520.19788
(M+Na)_{fnd}=520.19780
H¹NMR: CD3OD; δ 0.10 (s, 6H, Si-Me₂), 0.94 (s, 9H, Si-tBut), 3.40-3.62 (m, 3H, Glu 2,3,4-H), 4.00 (d, J=9.5Hz, Glu 5-H), 4.66-4.70 (m, 4H, OCH₂-C=C and ArCH₂), 4.8 (s, Glu 2,3,4-OH), 5.20-5.25 (m, 1H, allyl), 5.33-5.40 (m, 1H, allyl), 5.50 (d, 1H, J=7.7Hz, Glu-1H), 5.89-6.02 (m, 1H, allyl), 7.25 (d, 2H, J=2.9Hz, Ar), 7.41 (d, 2H, J=2.9Hz, Ar)

### Example 3.

### Synthesis of allyl 3,4,5-trihydroxy-6-([4-(hydroxymethyl)aniino]carbonyloxy)tetrahydro-2H-2 pyrancarboxylate 10b from allyl 3,4,5,6-tetrahydrotetrahydro-2H-2-pyrancarboxylate 9b (scheme 4).

To *4-([1-(tert-butyl)-1,1-dimethylsilyl]oxymethyl)phenyl isocyanate* **3** prepared from 2 g (7.52 mmol) of HOOC-Ph-CH₂-OTBDMS as described under example 2, was added 1.32 g (5.65 mmol) of **9b** dissolved in 15 ml of acetonitrile. The mixture was stirred overnight and then acidified with 0.5N KHSO₄ solution. After removal of the solvents in vacuo the residue was purified by flash chromatography (3% methanol/ethyl acetate followed by 5% methanol/ethylacetate).
Yield: 0.6g (31%) of **10b.**
(M+Na)_{cal=}406.11140
(M+Na)_{fnd}=406.11018
H¹NMR: CD₃OD; δ 3.40-3.47 (m, 3H, Glu 2,3,4-H), 3.99 (d, 1H, J=9.5Hz, Glu 5-H), 4.54 (s, 2H, 2H, Ar-CH₂), 4.67-4.70 (m, 2H, -OCH₂-C=C), 4.84 (s, Glu 2,3,4-OH, ArC-OH), 5.20-5.25 (m, 1H, allyl), 5.33-5.44 (m, 1H, allyl), 5.49 (d, 1H, J=7.6Hz, Glu 1-H), 5.91-6.00 (m, 1H, allyl), 7.27 (d, 2H, J=8.7Hz, Ar), 7.42 (d, 2H, J=8.4, Ar)

### Example 4.

### Synthesis of DOX-alGA3 1 (R¹=OMe, R²=OH, R=allyl) from allyl 3,4,5,6-tetrahydroxytetrahydro-2H-2-pyrancarboxylate 10b (scheme 4).

In a flame dried reaction vessel was dissolved 100 mg (0.26 mmol) of **10b** in 5 ml of acetonitrile. To this solution was added 56 mg (0.28 mmol) of 4-nitrophenylchloroformate and 25 mg (0.32 mmol) of pyridine. After 30 min at room temperature again 28 mg(0.14mmol) of 4-nitrophenyl- chloroformate and 13 mg (0.16mmol) of pyridine were added. After 1h at room temperature TLC showed that the starting compound had nearly disappeared. The reaction mixture was concentrated in vacuo and the residue was purified by column chromatography (8% methanol/dichloromethane).

Yield: 58 mg of *allyl 6-([4-([(4-chlorophenoxy)carbonyl]oxymethyl)anilino]carbonyloxy)-3,4,5-trihydroxytetrahydro-2H-2-pyrancarboxylate* **11b** (41%).
(M+Na)_{cal}= 571.11761
(M+Na)_{fnd}=571.11728
H¹NMR: CD₃OD; δ 3.40-3.61 (m, 3H, Glu 2,3,4-H), 3.99 (d, 1H, J=9.5Hz, Glu 5-H), 4.67-4.70 (m, 2H, -OCH₂-C=C), 4.84 (s, 4H, Glu 2,3,4-OH, ArC-OH), 5.20-5.25 (m, 3H, allyl and ArCH₂), 5.33-5.40 (m, 1H, allyl), 5.52 (d, 1H, J=7.6Hz, Glu 1-H), 5.89-6.02 (m, 1H, allyl), 7.38-7.52 (m, 6H, Ar), 8.30 (m, 2H, Ar-NO₂)

In a flame dried reaction vessel was dissolved 55 mg (0.1 mmol) of **11b** in 3 ml of dry DMF. To this solution was added 58 mg (0.1 mmol) of DOX.HCl and 10.1 mg (0.1 mmol) of triethyl amine. The reaction mixture was stirred overnight under an argon atmosphere and after that the solvent was removed in vacuo and the residue was purified by column chromatography (10% methanol/dichloromethane followed by 15% methanol/dichloromethane).
Yield: 72 mg (75%) of DOX-alGA3 **1** (R¹=OMe, R²=OH, R=allyl).
(M+Na)_{cal}=975.26472
(M+Na)_{fnd}=975.26785
H¹NMR: (CD₃)₂SO; δ 1.12 (d, 3H, J=6,6Hz, 5'-CH₃), 1.47 (bdd, 1H, 2'eq-H), 1.78-1.88 (m, 1H, 2'ax-H), 2.08-2.23 (m, 2H, 8ax-and 8eq-H), 2.90-3.04 (bAB pattern, 2H, 10eq- and 10ax-H), 3.15-3.50 (m, 4H, 4'-H, Glu 2,3,4-H), 3.64-3.78 (m, 1H, 3'-H), 3.91 (d, 1H, J=9.2Hz, Glu 5-H), 3.99 (s, 3H, 4-OMe), 4.15 (q, 1H, J=6.3Hz, 5'-H), 4.57 (d, 2H, J=6.1Hz, 14-CH₂), 4.61 (d, 2H, J=5.5Hz, -OCH₂-C=C), 4.68 (d, 1H, J=5.3Hz, 4'-OH), 4.83 (t, 1H, J=5.8Hz, 14-OH), 4.88 (s, 2H, ArCH₂), 4.94 (bt, 1H, 7-H), 5.18-5.45 (m, 8H, 1'-H, Glu1-H, 2x allyl, 9-OH, Glu 2,3,4-OH), 5.86-5.92 (m, 1H, allyl), 6.83 (d, 1H, J=7.9Hz, 3'-NH), 7.24 (d, 2H, J=8.4Hz, Ar 3,5-H), 7.42 (d, 2H, J=8.4Hz, Ar 2,6-H), 7.62-7.66 (m, 1H, 3-H), 7.90-7.92 (m, 2H, 1,2-H), 9.97 (s, 1H, Ar N-H), 13.25 (bs, 1H, 11-OH), 14.09 (bs, 1H, 6-OH)

### Example 5.

### Synthesis of the propargyl glucuronide spacer moiety allyl 3,4,5-tri(acetyloxy)-6-([4-([1-(text-butyl)-1,1-dimethylsilyl]oxymethyl)anilino]carbonyloxy)tetrahydro-2H 2-pyrancarboxylate 5b from allyl 3,4,5,6-tetrahydroxytetrahydro-2H-2-pyrancarboxylate 9b (scheme 5).

In 25 ml of pyridine was dissolved 2 g (8.55 mmol) of **9b** and to the solution 25 ml of acetic anhydride were added. The mixture was left overnight in a refrigerator and then concentrated at 30°C/0.8mm. The residue was purified by column chromatography (heptane/ ethylacetate=7/3).
Yield: 3g (87%) of *allyl 3,4,5,6-tetra(acetyloxy)tetrahydro-2H-2-pyrancarboxylate* **12b**.

Mixture of a and β isomers, NMR of β isomer identical with litt. (M. Tosin et all, J. Org. Chem. 2005, 70, 4096-4106). H¹NMR: CDCl₃ α- isomer; 5 4.44 (d, 1H, J=9.2Hz, Glu 1-H), 6.40 (d, 1H, J=1.2Hz, Glu 1-H)

In dry DMF was dissolved 3 g (7.46 mmol) of **12b** and to the solution was added 0.7 g of ammonium carbonate. After stirring for 40h at room temperature the mixture was acidified with 20ml of 0.5N KHSO₄ solution and 20 ml of water were added. The mixture was extracted three times with 30 ml of dichloromethane and the dichloromethane layer was subsequently washed three times with 30 ml of a KHSO₄ solution followed by two times washings with 30 ml of brine and than dried over sodium sulphate. After evaporation of the solvent the residue was purified by column chromatography (heptane/ethyl acetate =3/2). Yield: 1.56 g (58%) of *allyl 3,4,'5-tri(acetyloxy)-6-hydroxytetrahydro-2H-2-pyrancarboxylate* **4b**.
(M+Na)_{cal}=383.09542
(M+Na)_{fnd}=383.09448
H¹NMR: CDCl₃; δ 2.01 (s, 3H, OAc), 2.03 (s, 3H, OAc), 2.08 (s, 3H, OAc), 3.98 (bs, 1H, 1-OH), 4.55-4.69 (m, 3H, Glu 5-H, -OCH₂-C=C), 4.91 (dd, 1H, J=10.0Hz, 3.6Hz, Glu 2-H), 5.16-5.39 (m, 3H, Glu 4-H, allyl), 5.54-5.62 (m, 2H, Glu 1,3-H), 5.82-5.96 (m, 1H, allyl)

To *4-([1-(tert-butyl)-1,1-dimethylsilyl]oxymethyl)phenyl isocyanate* **3** prepared from 4.65 g (17.5 mmol) of HOOC-Ph-CH₂-OTBDMS as described under example 2, was added 4.72 g (31.1 mmol) of **4b** . After stirring for 18 h the mixture was diluted with 300 ml of diethyl ether and washed three times with 60 ml of 0.5N KHSO₄ followed by washings with 60 ml of a saturated solution of NaHCO₃ (3 times) and brine (2 times). The organic layer was dried over sodium sulphate and then removed in vacuo.
The residue was purified by column chromatography (heptane/ethyl acetate =5/2).
Yield: 6.29 g (77%) of **5b**.
(M+Na)_{cal}=646.22957
(M+Na)_{fnd}=646.22639
H¹NMR: CDCl₃; δ 0.08 (s, 6H, Si-Me₂), 0.93 (s, 9H, Si-tBut), 2.02 (s, 3H, OAc), 2.04 (s, 3H, OAc), 2.06 (s, 3H, OAc), 4.22 (d, 1H, J=9.7Hz, Glu 5-H), 4.53-4.67 (m, 2H, -OCH₂-C=C), 4.70 (s, 2H, ArCH₂), 5.17-5.36 (m, 5H, Glu 2,3,4=H, allyl), 5.80 (d, 1H, J=8.0Hz, Glu 1-H), 5.81-5.94 (m, 1H, allyl), 6.83 (bs, 1H, NH), 7.25-7.37 (m, 4H, Ar)

### Example 6.

### Synthesis of the propargyl glucuronide spacermoiety 2-propynyl 3,4,5-trihydroxy-6-([4-(hydroxymethyl)anilino] carbonyloxy)tetrahydro-2H-2-pyrancarboxylate 10c from allyl 3,4,5-tri(acetyloxy)-6-([4-([1-(tert-butyl)-1,1-dimethylsilyl]oxymethyl)anilino] carbonyloxy) tetrahydro-2H-2-pyrancarboxylate 5b.

To 25 ml of dried propargyl alcohol in a flame dried vessel was added 20 mg (0.087 mmol) of sodium. To this alcoholate solution was added 0.5 g (0.8 mmol) of **5b** and after stirring the mixture for 1.5h it was acidified with 0.16 g (1.2 mmol) of KHSO₄ in 8ml of water. After evaporation of the solvents at 35°C/0.8mm the residue was purified by column chromatography (6% methanol/dichloromethane followed by 12%
methanol/dichloromethane).
Yield: 156 mg (51%) of **10c**.
(M⁺Na)_{calc}=404. 09575
(M+Na)_{fnd}=404.09449
H¹NMR: CD₃OD; δ 2.94 (t, J=2.5Hz, -C≡CH), 3.40-3.61 (m, 3H, Glu 2,3,4-H ), 4.01 (d, 1H, J=9.5Hz, Glu 5-H), 4.54 (s, 2H, ArCH₂), 4.78 (d, 1H, J=2.6Hz, -OCH₂-C≡C), 4.79 (d, 1H, J=2.3Hz, -OCH₂-C≡C), 4.85 (s, Glu 2,3,4-OH, Ar-OH), 5.51 (d, 1H, J=7.9Hz, Glu-1H), 7.29 (d, 2H, J=8.9Hz, Ar), 7.42 (d, 2H, J=8.5Hz, Ar)

### Example 7.

### Synthesis of DOX-propGA3 1 (R¹=OMe, R²= OH, R= propargyl) from 2-propynyl 3,4,5-trihydroxy-6-([4-(hydroxymethyl)anilino] carbonyloxy)tetrahydro-2H-2-pyrancarboxylate 10c.

Compound **10c** was dried by repeatedly dissolving in acetonitrile/toluene and removal of the solvents in vacuo. To 100 mg (0.26mmol) of dried **10c** dissolved in 5 ml of dry acetonitrile, 56 mg (0.28 mmol) of 4-nitrophenyl chloroformate and 25 mg (0.32 mmol) of pyridine were added. After stirring for 30 min at room temperature again 28 mg (0.14 mmol) of 4-nitrophenyl chloroformate and 13 mg (0.16 mmol) of pyridine were added. After continued stirring for 1h at room temperature the mixture was concentrated in vacuo and the residue was purified by column chromatography (8% methanol/dichloromethane).
Yield: 74 mg (52%) of *2-propynyl 6-([4-([(4-nitrophenoxy)carbonyl]oxymethyl)anilino] carbonyloxy)-3,4,5-trihydroxytetrahydro-2H-2-pyrancarboxylate* **11c**.
M_{cal}=569.10196
M_{fnd}=569.10173
H¹NMR: CD₃OD; δ 2.95 (t, J=2.5Hz, -C≡CH), 3.40-3.61 (m, 3H, Glu 2,3,4-H ), 4.01 (d, 1H, J=9.5Hz, Glu 5-H), 4.54 (s, 2H, ArCH₂), 4.78 (d, 1H, J=2.6Hz, -OCH₂-C≡C), 4.79 (d, 1H, J=2.6Hz, -OCH₂-C≡C), 4.84 (s, Glu 2,3,4-OH, Ar-OH), 5.25 (s, 2H, ArCH₂), 5.52 (d, 1H, J=7.9Hz, Glu-1H), 7.39-7.52 (m, 6H, Ar), 8.27-8.33 (m, 2H, Ar-NO₂)

### DOX-proparGA3 1 (R¹=OMe, R²=OH, R= propargyl):

To 70 mg (0.13 mmol) of **11c** in 5 ml of dry DMF were added 74 mg (0.13 mmol) of DOX. HCl and 12.9 mg (0.13 mmol) of triethylamine. The mixture was stirred overnight at room temperature under an argon atmosphere and than concentrated in vacuo. The residue was purified by chromatography (10% methanol/dichloromethane followed by 15%
methanol/dichloromethane).
Yield: 96 mg (79%) of DOX-proparGA3 **1** ( R¹=OMe, R²=OH, R=propargyl).
(M+Na)_{cal}=973.24907
(M+Na)_{fnd}=973.24885
H¹NMR: (CD₃)₂SO; δ 1.11 (d, 3H, J=6,6Hz, 5'-CH₃), 1.47 (bdd, 1H, 2'eq-H), 1.78-1.90 (m, 1H, 2'ax-H), 2.05-2.26 (m, 2H, 8ax- and 8eq-H), 2.90-3.04 (bAB.pattern, 2H, 10eq- and 10ax-H), 3.15-3.50 (m, 4H, 4'-H, Glu 2,3,4-H), 3.57 (t, 1H, J=2.6Hz, - C≡CH), 3.64-3.78 (m, 1H, 3'-H), 3.94 (d, 1H, J=9.6Hz, Glu 5-H), 3.99 (s, 3H, 4-OMe), 4.15 (q, 1H, J=6.3Hz, 5'-H), 4.57 (d, 2H, J=6.1Hz, 14-CH₂), 4.68 (d, 1H, J=5.3Hz, 4'-OH), 4.75 (t, 2H, J=2.6Hz, -OCH₂-C≡C, 4.83 (t, 1H, J=5.8Hz, 14-OH), 4.88 (s, 2H, ArCH₂), 4.94 (bt, 1H, 7-H), 5.20 (d, J=2.9Hz, 1'-H), 5.32 (d, J=4.8Hz, Glu-OH), 5.40-5.47 (m, 4H, Glu1-H, 9-OH, 2x Glu OH), 6.83 (d, 1H, J=7.9Hz, 3'-NH), 7.24 (d, 2H, J=8.4Hz, Ar 3,5-H), 7.42 (d, 2H, J=8.4Hz, Ar 2,6-H), 7.62-7.66 (m, 1H, 3-H), 7.90-7.92 (m, 2H, 1,2-H), 9.97 (s,1H, Ar N-H), 13.25 (bs, 1H, 11-OH), 14.09 (bs, 1H, 6-OH)

In the same way DAU-proparGA3 **1** R¹=OMe, R²=H, R= propargyl) was prepared from **11c** and DAU.HCl.
Eluens (10% methanol/dichloromethane).
Yield: 74% DAU-proparGA3 **1** ( R¹=OMe, R²=H, R=propargyl).
(M+Na)_{cal}=957.25416
(M+Na)_{fnd}=957.25342
H¹NMR: (CD₃)₂SO; δ 1.12 (d, 3H, J=6,6Hz, 5'-CH₃), 1.47 (bdd, 1H, 2'eq-H), 1.78-1.90 (m, 1H, 2'ax-H), 2.05-2.26 (m, 2H, 8ax- and 8eq-H), 2.26 (s, 3H, 13-CH₃), 2.89-3.02 (bAB pattern, 2H, 10eq- and 10ax-H), 3.15-3.50 (m, 4H, 4'-H, Glu 2,3,4-H), 3.57 (t, 1H, J=2.6Hz, -C≡CH), 3.64-3.78 (m, 1H, 3'-H), 3.94 (d, 1H, J=9.6Hz, Glu 5-H), 3.99 (s, 3H, 4-OMe), 4.15 (bq, 1H, J=6.3Hz, 5'-H), 4.68 (d, 1H, J=5.3Hz, 4'-OH), 4.75 (t, 2H, J=2.6Hz, - OCH₂-C≡C), 4.88 (s, 2H, ArCH₂), 4.94 (bt, 1H, J=4.8Hz, 7-H), 5.21 (d, J=2.5Hz, 1'-H), 5.32 (d, J=4.8Hz, Glu-OH), 5.39-5.47 (m, 3H, Glu 1-H, 2xGlu-OH), 5.53 (s, 1H, 9-OH), 6.83 (d, 1H, J=7.7Hz, 3'-NH), 7.24 (d, 2H, J=8.4Hz, Ar 3,5-H), 7.42 (d, 2H, J=8.4Hz, Ar 2,6-H), 7.62-7.66 (m, 1H, 3-H), 7.90-7.92 (m, 2H, 1,2-H), 9.97 (s,1H, Ar N-H), 13.29 (s, 1H, 11-OH), 14.03 (s, 1H, 6-OH)

### Example 8.

### Conversion of propargyl esters 1 (R¹=OMe, R²= OH, R=propargyl) into the corresponding triazol esters 1 (R¹=OMe, R²=OH, n=4 and 7, x=1) scheme 3.

Conversion of Me(OCH₂CH₂)ₙOH into Me(OCH₂CH₂)ₙN₃.

The methoxy polyethyleneglycols were not available as pure compounds but they were always accompanied by mixtures of the n+(1-3) and n-(1-3) derivatives.

We therefore distilled the commercial available compound for n=7 and isolated fractions with n=4 and n=7 which contained more than 90% of the main compound. The compound with n=12 was used as the commercial mixture.

General procedure: To 8 mmol of dried Me(OCH₂CH₂)ₙOH dissolved in 2 ml of pyridine was added 3.8 g (20mmol) of tosylchloride. The mixture was stirred overnight and after that poured into ice. The whole was extracted with dichloromethane and the dichloromethane solution was twice washed with 6N HCl and with water. After drying over magnesium sulfate the dichloromethane was evaporated and the residue was used without further purification. In this way were prepared Me(OCH₂CH₂)ₙN₃ with n=4, 7 and 12.

To 70 mg (0.074 mmol) of DOX-proparGA3 dissolved in 2.5 ml of THF/Water (4/1) was added 70 mg (4eq) of Me(OCH₂CH₂)₄N₃, 3 mg (25 mol%) of CuSO₄ and 7.3 mg (50 mol%) of sodium ascorbate. After stirring overnight under an argon atmosphere the mixture was concentrated at 35°/0.8mm and the residue was several times washed with diethyl ether to remove the excess azide. The residue was purified by column chromatography (eluens 10% methanol/dichloromethane followed by 15%
methanol/dichloromethane).
Yield: 65 mg (73%) of triazol ester **1** ( R¹=OMe, R²=OH, n=4, x=1)
(M+Na)_{calc}=1206.38663
(M+Na)_{fnd}=1206.380.08
H¹NMR: (CD₃)₂SO; δ 1.12 (d, 3H, J=6,6Hz, 5'-CH₃), 1.47 (bdd, 1H, 2'eq-H), 1.78-1.88 (m, 1H, 2'ax-H), 2.08-2.23 (m, 2H, 8ax- and 8eq-H), 2.90-3.04 (b AB pattern, 2H, 10eq- and 10ax-H), 3.17-3.52 (m, 18H, 4'-H, Glu 2,3,4-H, (O-C-C)₄), 3.31 (s, 3H, OCH₃), 3.64-3.78 (m, 1H, 3'-H), 3.79 (t, 2H, J=5.1Hz, CH₂ triazyl), 3.89 (d, 1H, J=9.1 Hz, Glu 5-H), 3.99 (s, 3H, 4-OMe), 4.15 (q, 1H, J=5.9Hz, 5'-H), 4.51 (t, 2H, J=5.4Hz, COOCH₂), 4.57 (d, 2H, J=5.9Hz, 14-CH₂), 4.68 (d, 1H, J=5.8Hz, 4'-OH), 4.83 (t, 1H, J=5.9Hz, 14-OH), 4.87 (s, 2H, ArCH₂), 4.93-4.98 (m, 1H, 7-H), 5.17-5.24 (m, 2H, 1'-H, Glu-OH), 5.31 (d, 1H, J=4.8Hz, Glu-OH), 5.37-5.48 (m, 3H, Glu1-H, 9-OH, Glu-OH), 6.82 (d, 1H, J=8.0Hz, 3'-NH), 7.24 (d, 2H, J=8.4Hz, Ar 3,5-H), 7.41 (d, 2H, J=8.4Hz, Ar 2,6-H), 7.62-7.66 (m, 1H, 3-H), 7.89-7.95 (m, 2H, 1,2-H), 8.12 (s, 1H, triazyl), 9.96 (s,1H, Ar N-H), 13.28 (bs, 1H, 11-OH), 14.04 (bs, 1H, 6-OH)

In the same way were prepared :
Triazol ester **1** ( R¹=OMe, R²=OH, n=7, x=1)
Eluens (5% methanol/dichlormethane followed by 12% methanol/dichloromethane).
Yield: 51%
(M+Na)_{calc}=1338.4627
(M+Na)_{fnd}=1338.4565
H¹NMR: (CD₃)₂SO; δ 1.11 (d, 3H, J=6,6Hz, 5'-CH₃), 1.47 (bdd, 1H, 2'eq-H), 1.77-1.90 (m, 1H, 2'ax-H), 2.08-2.23 (m, 2H, 8ax- and 8eq-H), 2.90-3.04 (bAB pattern, 2H, 10eq- and 10ax-H), 3.18-3.52 (m, 30H, 4'-H, Glu 2,3,4-H, (-O-CH₂CH₂)₇), 3.64-3.78 (m, 1H, 3'-H), 3.79 (t, 2H, J=5.1Hz, CH₂ triazyl), 3.89 (d, 1H, J=9.2 Hz, Glu 5-H), 3.99 (s, 3H, 4-OMe), 4.15 (q, 1H, J=6.9Hz, 5'-H), 4.51 (t, 2H, J=5.2Hz, COOCH₂), 4.57 (d, 2H, J=5.9Hz, 14-CH₂), 4.68 (d, 1H, J=5.5Hz, 4'-OH), 4.83 (t, 1H, J=5.9Hz, 14-OH), 4.87 (s, 2H, ArCH₂), 4.95 (t, 1H, 7-H), 5.17-5.24 (m, 2H 1'-H, Glu-OH), 5.31 (d, 1H, J=4.1Hz, Glu-OH), 5.37-5.48 (m, 3H, Glu1-H, Glu-OH, 9-OH), 6.82 (d, 1H, J=8.4Hz, 3'-NH), 7.24 (d, 2H, J=8.4Hz, Ar 3,5-H), 7.41 (d, 2H, J=8.0Hz, Ar 2,6-H), 7.62-7.68 (m, 1H, 3-H), 7.89-7.95 (m, 2H, 1,2-H), 8.11 (s, 1H, triazyl), 9.96 (s,1H, Ar N-H), 13.28 (bs, 1H, 11-OH), 14.04 (bs, 1H, 6-OH)

Triazol ester **1** (R¹=OMe, R²=H, n=4, x=1)
Eluens (10% methanol/dichloromethane).
Yield: 69%
(M+Na)_{calc}=1190.39172
(M+Na)_{fnd}=1190.40091
H¹NMR: (CD₃)₂SO; δ 1.12 (d, 3H, J=6,6Hz, 5'-CH₃), 1.45 (bdd, 1H, 2'eq-H), 1.78-1.90 (m, 1H, 2'ax-H), 2.05-2.26 (m, 2H, 8ax- and 8eq-H), 2.26 (s, 3H, 13-CH₃), 2.89-3.02 (bAB pattern, 2H, 10eq- and 10ax-H), 3.17-3.52 (m, 18H, 4'-H, Glu 2,3,4-H, (O-CH₂CH₂)₄), 3.66-3.78 (m, 1H, 3'-H), 3.79 (t, 2H, J=5.1Hz, CH2 triazyl), 3.89 (d, 1H, J=9.2Hz, Glu 5-H), 3.98 (s, 3H, 4-OMe), 4.16 (bq, 1H, J=6.3Hz, 5'-H), 4.51 (t, 2H, J=5.1Hz, COOCH₂), 4.68 (d, 1H, J=5.9Hz, 4'-OH), 4.88 (s, 2H, ArCH₂), 4.94 (bt, 1H, J=4.1Hz, 7-H), 5.12-5.25 (m, 2H, 1'-H, Glu-OH), 5.28-5.36 (m, Glu-OH), 5.39-5.47 (m, 2H, Glu-1H, Glu-OH), 5.53 (s, 1H, 9-OH), 6.81 (d, 1H, J=8.1Hz, 3'-NH), 7.24 (d, 2H, J=8.4Hz, Ar 3,5-H), 7.42 (d, 2H, J=8.4Hz, Ar 2,6-H), 7.62-7.66 (m, 1H, 3-H), 7.90-7.92 (m, 2H, 1,2-H), 8.12 (s, 1H, triazyl), 9.97 (s,1H, Ar N-H), 13.29 (bs, 1H, 11-OH), 14.03 (bs, 1H, 6-OH)

Triazol ester **1** (R¹=OMe, R²=H, n=7, x=1)
Eluens (10% methanol/dichloromethane).
Yield: 52%
(M+Na)_{calc}=1322.47036
(M+Na)_{fnd}=1322.46150
H¹NMR: (CD₃)₂SO; δ 1.12 (d, 3H, J=6,6Hz, 5'-CH₃), 1.45 (bdd, 1H, 2'eq-H), 1.78-1.90 (m, 1H, 2'ax-H), 2.05-2.26 (m, 2H, 8ax- and 8eq-H), 2.26 (s, 3H, 13-CH₃); 2.89-3.02 (bAB pattern, 2H, 10eq- and 10ax-H), 3.18-3.52 (m, 30H, 4'-H, Glu 2,3,4-H, (-O-CH₂CH₂)₇), 3.66-3.78 (m, 1H, 3'-H), 3.79 (t, 2H, J=5.2Hz, CH₂ triazyl), 3.89 (d, 1H, J=9.2Hz, Glu 5-H), 3.98 (s, 3H, 4-OMe), 4.16 (bq, 1H, J=6.3Hz, 5'-H), 4.51 (t, 2H, J=5.4Hz, COOCH₂), 4.68 (d, 1H, J=5.9Hz, 4'-OH), 4.88 (s, 2H, ArCH₂), 4.93 (bt, 1H, J=4.0Hz, 7-H), 5.12-5.25 (m, 2H, 1'-H, Glu-OH), 5.32 (d, J=4.8Hz, Glu-OH), 5.38-5.47 (m, 2H, Glu 1-H, Glu-OH), 5.53 (s, 1H, Glu-OH), 6.81 (d, 1H, J=8.1Hz, 3'-NH), 7.24 (d, 2H, J=8.4Hz, Ar 3,5-H), 7.42 (d, 2H, J=8.4Hz, Ar 2,6-H), 7.62-7.66 (m, 1H, 3-H) , 7.90-7.92 (m, 2H, 1,2-H), 8.12 (s, 1H, triazyl), 9.97 (s,1H, Ar N-H), 13.29 (bs, 1H, 11-OH), 14.03 (bs, 1H, 6-OH)

Triazol ester **1** (R¹=OMe, R²=OH, n=12, x=1)
Contained only for about 60% of the derivative with n=12.
Eluens (10% methanol/dichloromethane followed by 15%
methanol/dichloromethane).
Yield: 46%
(M+Na)_{calc}=1558.59635
(M+Na)_{fnd}=1558.59630

### Example 9.

### Synthesis of the propargyl glucuronide di-spacermoiety 2-propynyl 3,4,5-trihydroxy-6-[(4-[([4-(hydroxymethyl)anilino]carbonyloxy)methyl]anilinocarbonyl)oxy] tetrahydro-2H-2-pyrancarboxylate 15c from allyl 3,4,5-tri(acetyloxy)-6-([4-(hydroxymethyl)anilino] carbonyloxy)tetrahydro-2H-2-pyrancarboxylate 6b.

2 g (3.2mmol) *allyl 3,4,5-tri(acetyloxy)-6-([4-([1-(tert-butyl)-1,1-dimethylsilyl] oxymethyl)anilino] carbonyloxy) tetrahydro-2H-2-pyrancarboxylate* **5b** was hydrolyzed in 90 ml tetrahydrofuran/water/acetic acid=1/1/1. After 4 hrs the reaction mixture was extracted with 50 ml dichlormethane (3 times). The dichloromethane layer was subsequently washed with 30 ml of water (2 times) followed by 30 ml of a saturated sodium bicarbonate solution (2 times) and by 30 ml of brine (2 times). The organic layer was dried over sodium sulphate and then removed in vacuo. The residue was purified by column chromatography (heptane/ ethylacetate=1/1).
Yield: 1.45 gr (89%) of **6b**
(M+Na)_{cal}=532.14309
(M+Na)_{fud}=532.14142
H¹NMR: CDCl₃; δ 2.02 (s, 3H, OAc), 2.04 (s, 3H, OAc), 2.05 (s, 3H, OAc), 4.22 (d, 1H, J=8.5Hz, Glu 5-H), 4.53-4.67 (m, 4H, - OCH₂-C=C, ArCH₂), 5.13-5.37 (m, 5H, Glu 2,3,4-H, allyl), 5.76 (d, 1H, J=8.0Hz, Glu 1-H), 5.81-5.91 (m, 1H, allyl), 7.05 (bs, 1H, NH), 7.26-7.39 (m, 4H, Ar)

To *4-([1-(tert-butyl)-1,1-dimethylsilyl]oxymethyl)phenyl isocyanate* **3** prepared from 0.8 g (3 mmol) of HOOC-Ph-CH₂-OTBDMS as described under example 2, was added 1 g (2 mmol) of **6b.** After stirring for 18 h the mixture was concentrated in vacuo and the residu was purified by column chromatography (heptane/ethyl acetate =2/1).
Yield: 1 g (66%) of *allyl 3,4,5-tri(acetyloxy)-6-([4-([1-(tert-butyl)-1,1-dimethylsilyl]*
*oxymethyl)anilino]carbonyloxy)tetrahydro-2H-2-pyrancarboxylate* **14b**.
(M+Na)_{cal}=795.27725
(M+Na)_{fnd}=795.27285
H¹NMR: CDCl₃; δ 0.08 (s, 6H, Si-Me₂), 0.93 (s, 9H, Si-tBut), 2.02 (s, 3H, OAc), 2.04 (s, 3H, OAc), 2.05 (s, 3H, OAc), 4.22 (d, 1H, J=9.5Hz, Glu 5-H), 4.53-4.63 (m, 2H, -OCH₂-C=C), 4.68 (s, 2H, ArCH₂), 5.13 (s, 2H, ArCH₂), 5.13-5.39 (m, 5H, Glu 2,3,4-H, allyl), 5.78 (d, 1H, J=8.0Hz, Glu 1-H), 5.82-5.94 (m, 1H, allyl), 6.69 (bs, 1H, ArNH), 7.02 (bs, 1H, ArNH), 7.25 (d, 2H, J=8.7Hz, Ar), 7.31-7.40 (m, 6H, Ar)

To 25 ml of dried propargyl alcohol in a flame dried vessel was added 24 mg (1.1 mmol) of sodium. To this alcoholate solution was added 0.75 g (0.97 mmol) of **14b** and after stirring the mixture for 1.5h it was acidified with 0.17 g (1.2 mmol) of KHSO₄ in 10 ml of water. After evaporation of the solvents at 35°C/0.8mm the residue was purified by column chromatography (2% methanol/ethylacetate)
Yield: 230 mg (44%) of **15c.**
(M+Na)_{calc}=404.09575
(M+Na)_{fnd}=404.09449
H¹NMR: CD₃OD; δ 2.93 (t, J=2.3Hz, -C≡CH), 3.39-3.60 (m, 3H, Glu 2,3,4-H ), 4.00 (d, 1H, J=9.5Hz, Glu 5-H), 4.52 (s, 2H, ArCH₂-OH), 4.76-4.79 (m, 2H, -OCH₂-C≡C), 4.83 (s, Glu 2,3,4-OH, Ar-OH), 5.11 (s, 2H, ArCH₂), 5.50 (d, 1H, J=7.7Hz, Glu-1H), 7.25 (d, 2H, J=8.5Hz, ArCH₂), 7.24-7.42 (m, 6H, Ar)

### Example 10.

### Synthesis of 1 (R¹=OMe, R²=OH, R=propargyl, p=2) from 2-propynyl 3,4,3-trihydroxy-6-[(4-[([4 (hydroxymethyl)anilino]carbonyloxy)methyl]anilinocarbonyl)oxy] tetrahydro-2H-2-pyrancarboxylate 15c.

First *2-propynyl 6-[(4-[([4-([(4-nitrophenoxy)carbonyl]oxymethyl )anilino] carbonyloxy) methyl]anilinocarbonyl)oxy]-3,4,5-trihydroxytetrahydro-2H-2-pyrancarboxylate* **16c** was prepared from **15c** analogously as described for the conversion of **10c** into **11c**.
Eluens: (methanol/ethylacetate=1/99).
Yield: 50% of **16c**.
(M+Na)_{cal}=718.14964
(M+Na)_{fnd}=718.14820
H¹NMR: CD₃OD; δ 2.93 (t, J=2.6Hz; 1H, -C≡CH), 3.45 (m, 3H, Glu 2,3,4-H), 4.00 (d, J=9.5Hz, 1H, Glu 5-H), 4.75-4.80 (m, 2H, COOCH₂), 4.85 (s, Glu 2,3,4-OH), 5.12 (s, 2H, ArCH₂), 5.22 (s, 2H, ArCH₂), 5.50 (d, J=7.7Hz, 1H, Glu 1-H), 7.27-7.48 (m, 10H, Ar), 8.26-8.32 (m, 2H, Ar-NO₂)

The conversion of **16c** into **1** (R¹=OMe, R²=OH, R=propargyl, p=2) was carried out analogously to the conversion of **11c** into DOX-proparGA3 **1**.
Eluens: (10% methanol/dichloromethane followed by 15% methanol/dichloromethane).
Yield: 74% of **1** (R¹=OMe, R²=OH, R=propargyl, p=2)
(M+Na)_{calc}=1122.29675
(M+Na)_{fnd}=1122.29075
H¹NMR: (CD₃)₂SO; δ 1.11 (d, 3H, J=6,2Hz, 5'-CH₃), 1.47 (bdd, 1H, 2'eq-H), 1.78-1.90 (m, 1H, 2'ax-H), 2.05-2.26 (m, 2H, 8ax- and 8eq-H), 2.90-3.04 (bAB pattern, 2H, 10eq- and 10ax-H), 3.15-3.50 (m, 4H, 4'-H, Glu 2,3,4-H), 3.58 (t, J=2.4Hz, 1H, - C≡CH), 3.64-3.75 (m, 1H, 3'-H), 3.96 (d, J=8.0Hz, 1H, Glu 5-H), 3.96 (s, 3H, 4-OMe), 4.12 (bq, 1H, 5'-H), 4.57 (d, 2H, J=5.9Hz, 14-CH₂), 4.66 (d, 1H, J=5.5Hz, 4'-OH), 4.76 (t, J=2.6Hz, 2H, -OCH₂-C≡C, 4.78-4.88 (m, 3H, 14-OH, ArCH₂), 4.92 (bt, 1H, 7-H), 5.02 (bs, 2H, ArCH₂), 5.18 (bd, 1'-H), 5.30 (bd, Glu-OH), 5.39-5.47 (m, 4H, Glu1-H, 9-OH, 2x Glu OH), 6.81 (bd, 1H, 3'-NH), 7.21 (d, J=8.4Hz, 2H, Ar), 7.33-7.44 (m, 4H, Ar), 7.48 (d, J=8.8Hz, 2H, Ar), 7.62-7.66 (m, 1H, 3-H), 7.90 (d, J=4.4Hz, 2H, 1,2-H), 9.97 (s,1H, Ar N-H), 13.27 (bs, 1H, 11-OH), 14.02 (bs, 1H, 6-OH)

### Example 11.

### Hydrolysis of DOX-mGA3 and DOX-triazol esters 1 ( n=, 7 and 12, x=1).

The hydrolysis of the DOX esters were qualitatively followed with reverse phase TLC ( RP18). With eluens acetonitril/water: 42/58 in a phosphate buffer (KH₂PO₄/Na₂HPO₄) of pH=7.33. DOX-mGA3 0.29 mg was dissolved in 1 ml of buffer together with some drops of DMSO to keep the DOX-mGA3 dissolved. It took 24 hrs before the starting compound had disappeared.

After concentration of the water solution the remaining compound appeared to be DOX-GA3 from its mass spectrum.

In the same way the hydrolysis of DOX-triazol esters 1 (n=, 7 and 12, x=1) were followed for 0.35 mg/ml and 0.45 mg/ml buffer. Both esters were completely hydrolyzed within 4 hrs.

### Example 12

### Prodrug activation by GUS and FBS

In vitro antiproliferative effects in OVCAR-3 cells after 3 days of incubation with the different (pro)-drugs DOX, DOX-alGA3, DOX-mPEG4-GA3 and DOX-mPEG7-GA3 in the presence or absence of bovine beta-glucuronidase(GUS).

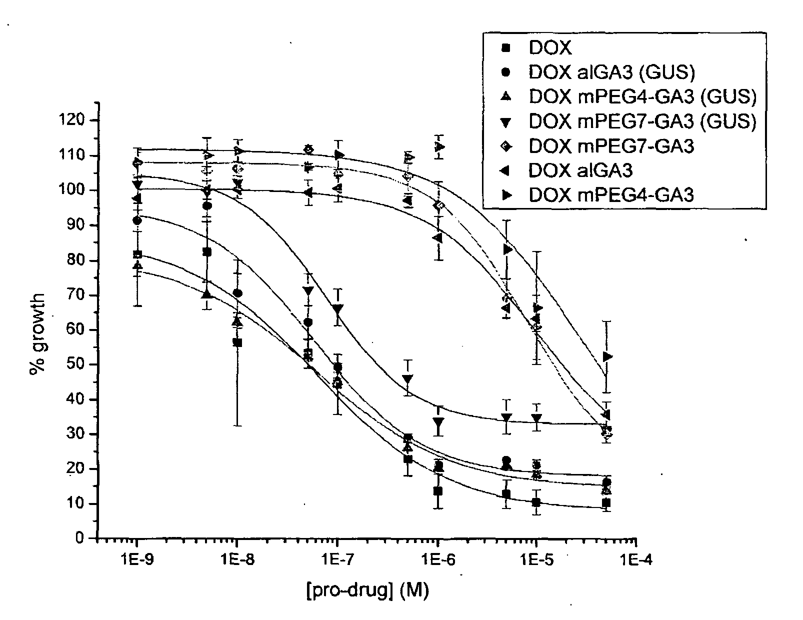

The concentrations used for the prodrugs ranged from 0.001 µM to 50 µM, the GUS enzyme was added to give a final concentration of 10 units/µl. Adjacent to this, cells treated with the prodrugs were incubated without GUS. Incubation was conducted at 37°C in a humidified, 5% CO₂ incubator of three days. Cell growth is measured by crystal violet assay, untreated cells are set at 100%. The IC₅₀ values found in these experiments are depicted in the table beneath.
IC50 (M)

| | No GUS | with GUS (10 units/µL) |
|---|---|---|
| DOX | 6.19E-08 | |
| DOX alGA3 | 9.81E-06 | 5.8.4E-08 |
| DOX mPEG4-GA3 | 2.21E-05 | 6.21E-08 |
| DOX mPEG7-GA3 | 8.33E-06 | 7.74E-08 |

The experimental conditions were as follows:

### Materials

Dulbecco's modified Eagle's medium (DMEM), Trypsin and penicillin/streptomycin (P/S) were purchased from Invitrogen. Fetal bovine serum (FBS) was purchased from PAA laboratories. β-Glucuronidase from bovine liver (GUS) purchased from Sigma Aldrich. Crystal violet was purchased from Fluka Chemika. Acetonitril and methanol was purchased from Biosolve and the ethanol was purchased from Merck

### Cell based anti-proliferative effects

The human ovarian cancer (OVCAR3) cell line was maintained in DMEM supplemented with 10% heat inactivated FBS and 1% P/S. Inhibition of proliferation of the OVCAR cell line by the doxorubicin prodrugs Dox-allyl, Dox-mGA3, Dox-mPEG4 and Dox-mPEG7 was determined using a crystal violet assay. The assay was preformed in a 96-wells plate. Cells that were harvested by trypsinisation were seeded with 5.000 cells in 100 µl medium per well. The 96-wells plate was incubated overnight at 37 °C in a humidified, 5% CO₂ incubator. The different prodrugs were added in concentrations between 0.001 µM to 50 µM. Subsequently, the GUS enzyme was added to give a final concentration of 10 units/ µl. This mixture was incubated at 37 °C in a humidified, 5% CO₂ incubator for tree days. Subsequently, the cells were stained and fixed with 50 µl 1% crystal violet in 70% ethanol. The cells were washed with water and resolubilized in 100 µl 1% SDS. After this the absorbance was read at 550 nm. The blank extinction values were determined on wells in which no cells were seeded were subtracted from the signals. Inhibition concentrations 50% (IC₅₀) were derived by plotting the inhibition of cell proliferation at different concentrations of the prodrug and non-linear curve fitting.

### Prodrug activation

The rate at which the prodrugs are converted into doxorubin was determined by HPLC analysis. The prodrug and GUS enzyme were mixed in FBS with final concentrations of 10 µM for the prodrug and 10 units/µl for the enzyme. The hydrolysis of the prodrugs by esterase present in the FBS was also studied. In this case the prodrugs (10 µM) were diluted in FBS 100 µL and incubated at 37 °C. The reaction was terminated at different time points (≤ 5h) by adding 300 µL ice cold Methanol. The samples were centrifuged for 5 minutes at 16.1 RCF. Samples were stored at -20 °C until further use. The supernatants were analyzed by HPLC.

### HPLC

The HPLC apparatus consisted of an autosampler, a pump and a fluorescence detector (excitation: 450 emission: 550). 10 µl of the samples was injected and loaded on a reverse phase column (Waters sunfire C18, particle size: 5 µm, 4.6 mm x 150 mm column). The mobile phase used was 66:34 phosphate buffer (pH 7, 0.01 M): acetonitril.
β -Glucuronidase

## Claims

1. Anthracycline prodrug esters having formula 1 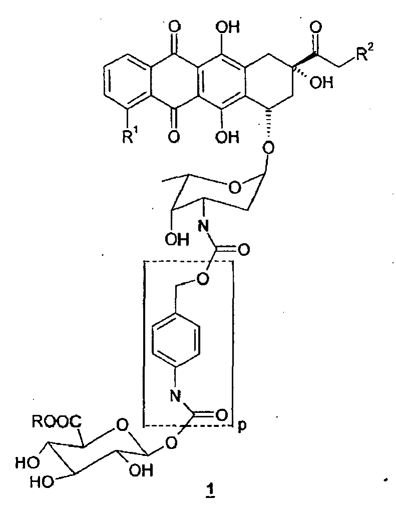 wherein:
R¹ = H or OCH₃;
R² = H or OH;
R = CH₂=CH-CH₂- ; CH≡C-(CH₂)ₓ- ; HO-CH₂-CH₂- ; CH₃O-CH₂-CH₂-; or a residue having formula 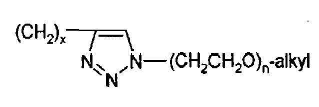
n is an integer from 1 to 40, preferably from 1 to 10, more preferably from 2 to 6;
p is an integer from 1 to 5, preferably from 1 to 3, more preferably 1 or 2;
x is an integer from 1 to 5, preferably from 1 to 3, more preferably 1 or 2;
alkyl is a straight or branched alkyl residue having 1-10 carbon atoms, preferably 1-3 carbon atoms.

2. A process for the preparation of anthracycline prodrug esters having formula 1 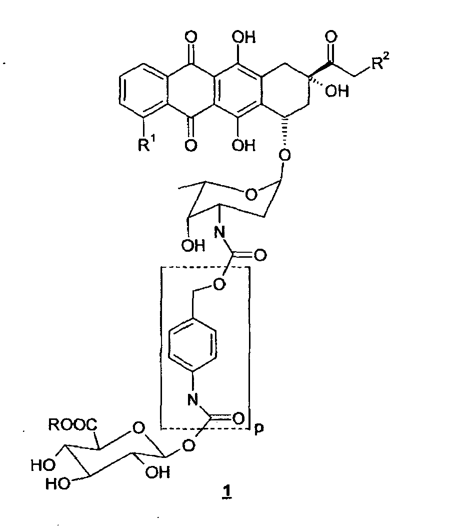 wherein:
R¹ = H or OCH₃;
R² = H or OH;
R = CH₃; CH₂=CH-CH₂- ; or CH≡C-(CH₂)ₓ- ;
comprising the reaction of a compound having formula 10a, 10b or 10c, having unprotected sugar hydroxyl groups, 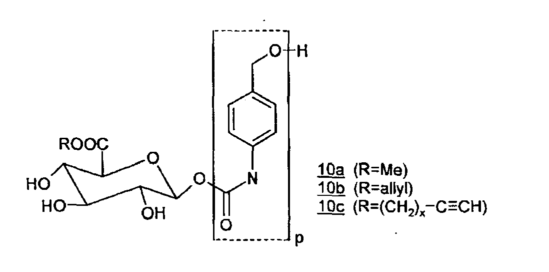 wherein p is an integer from 1 to 5, preferably 1 to 3, more preferably 1 or 2, with p-nitrophenylchloroformate, to obtain a compound having formula 11a, 11b or 11c respectively; 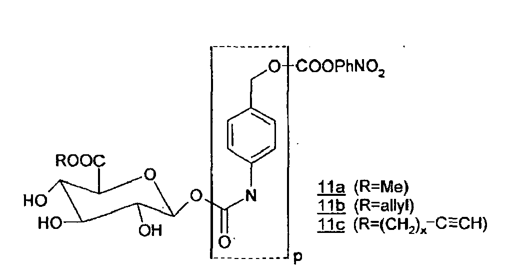 followed by the reaction with an anthracycline of formula 21 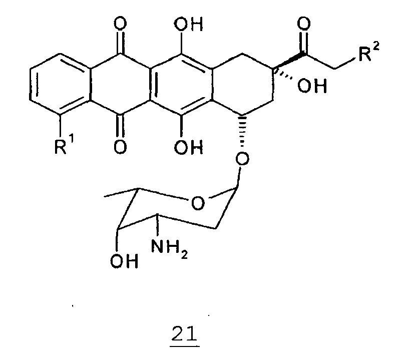 wherein R¹ and R² are as defined above, to obtain an anthracycline prodrug ester having a formula 1 wherein R is CH₃, allyl or (CH₂)ₓ-C=CH.

3. A process according to claim 2, wherein a compound having formula 10a or 10b, wherein p is 1, is prepared by reaction of an isocyanate having formula 3 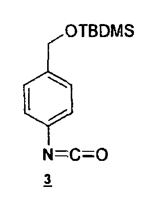 wherein TBDMS represents the t-butyldimethylsilyl protective group, with a completely unprotected glucuronic acid ester having formula 9a or 9b respectively, 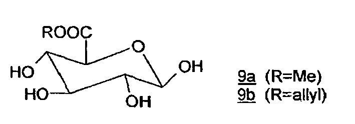 to obtain a compound having formula 13a or 13b respectively, 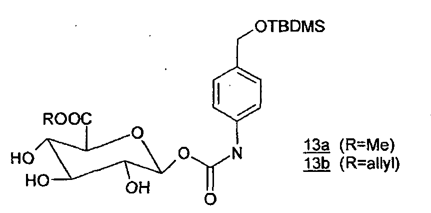 followed by deprotection of the silyl protective group to obtain a compound of formula 10a or 10b respectively.

4. A process according to claim 2, wherein a compound having formula 10c, wherein p is 1, 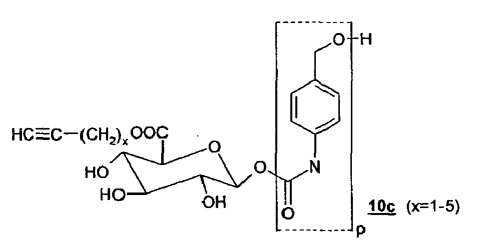 is prepared by reaction of a compound having formula 13a or 13b 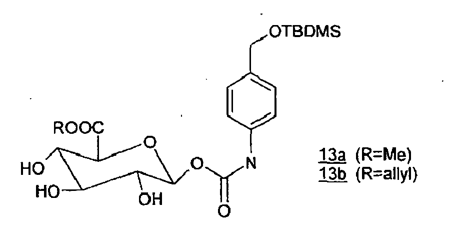 with an alcohol of formula CH≡C-(CH₂)ₓ-OH, wherein x is as defined in claim 1, in the presence of a base, followed by acidic removal of the silyl protective group, to obtain a compound of formula 10c.

5. A process for the preparation of a compound having formula 15a, 15b or 15c 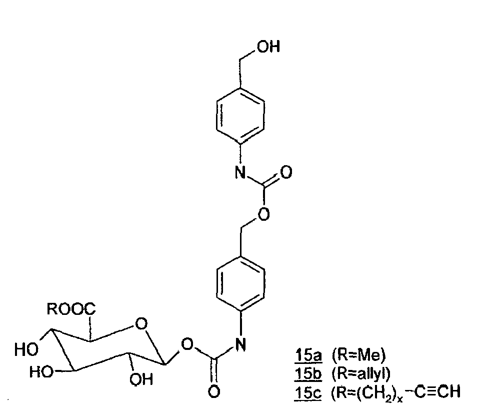 by reaction of a compound having formula 14a or 14b 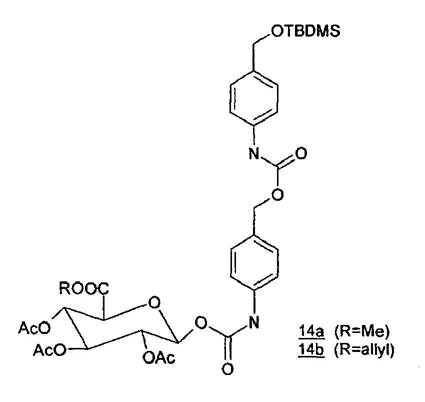 with an alcohol ROH, wherein R is Me, allyl or (CH₂)ₓ-C≡CH, in the presence of a base followed by removal of the silyl protective group to obtain a compound of formula 15a, 15b or 15c respectively.

6. A process for the preparation of an anthracycline prodrug ester having formula 1 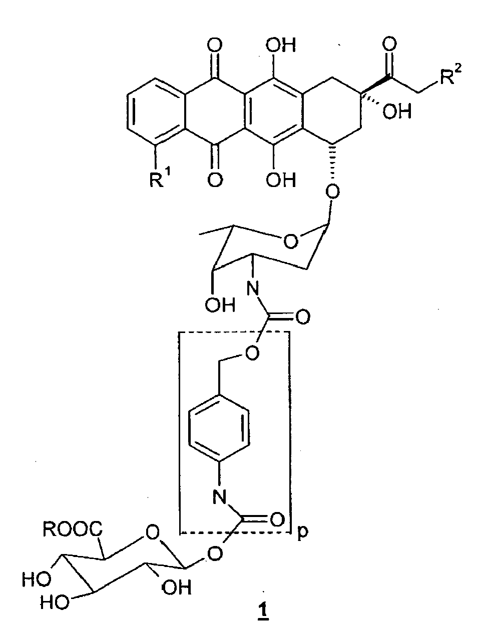 wherein:
R¹ = H, OCH₃ ;
R² = H, OH ;
R is a residue having formula: 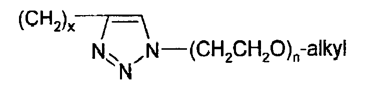
n is an integer from 1 to 40, preferably from 1 to 10, more preferably from 2 to 6;
p is an integer from 1 to 5, preferably from 1 to 3, more preferably 1 or 2;
x is an integer from 1 to 5, preferably from 1 to 3, more preferably 1 or 2;
alkyl is a straight or branched alkyl residue having 1-10 carbon atoms, preferably 1-3 carbon atoms;
by reaction of a compound having formula 1, wherein R is CH≡C-(CH₂)ₓ-, wherein R¹, R², p and x are as defined above, obtainable according to the process as defined in claim 2, with N₃-(CH₂CH₂O)ₙ-alkyl in the presence of a copper catalyst to obtain the corresponding mono-alkoxy-polyethyleneglycol triazyl esters of formula 1 wherein R¹, R², p, x and alkyl are as defined above, and alkoxy contains alkyl as defined above.

7. A process for the preparation of an anthracyline prodrug ester having formula 1. 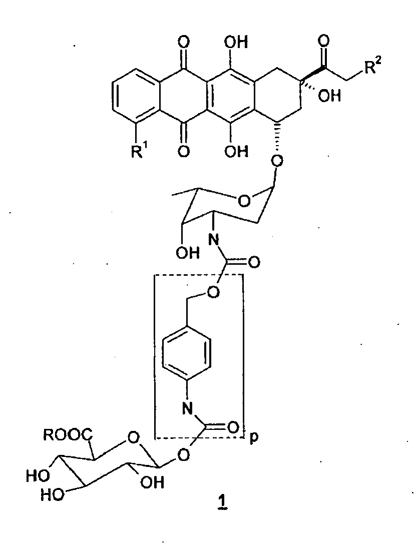
wherein:
R¹ = H or OCH₃;
R² = H or OH;
R = CH₂-CH₂OH, or CH₂-CH₂-OCH₃;
by reaction of a prodrug having formula 1, wherein R=CH₃, obtainable according to a process according to claim 2, with an alcohol of formula HO-CH₂-CH₂OH or HO-CH₂-CH₂-OCH₃ respectively, in the presence of a base, to obtain a prodrug ester having formula 2, wherein R= HO-CH₂-CH₂OH or HO-CH₂-CH₂-OCH₃ respectively.

8. Use of an anthracycline prodrug ester having formula 1, obtainable according to a process according to one or more of the claims 2 to 7, wherein R¹, R² and R are as defined in claims 2 to 7, in the preparation of a medicament for use in a target tissue treatment, wherein the ester is hydrolysed, and is thereafter selectively activated by an enzyme which is coupled to an antibody being specific for said target tissue.

9. Use according to claim 8, wherein said hydrolysed prodrug ester is activated by an endogeneous enzyme.

10. Use according to claim 8, wherein said hydrolysed prodrug ester is activated by an exogeneous enzyme.

11. Use according to claims 8 to 10, wherein said enzyme is β-glucuronidase.

12. Use according to claims 8 to 11, wherein the antibody has specificity for cancer cells.

13. An antitumour composition for administration orally, topically or by injection, containing as active ingredient an anthracycline derivative of formula 1. 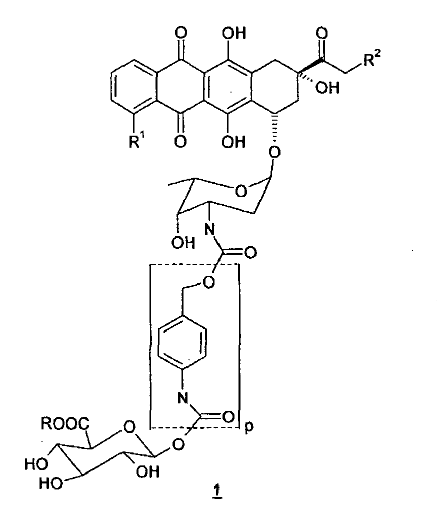 wherein:
R¹ = H, OCH₃;
R² = H, OH;
R = CH₂=CH-CH₂- ; CH≡C-(CH₂)ₓ- ; HO-CH₂-CH₂- ;
CH₃O-CH₂-CH₂- _{;} or a residue having formula: 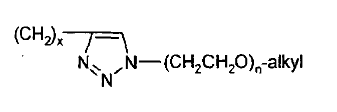
n is an integer from 1 to 40, preferably from 1 to 10, more preferably from 1 to 6;
p is an integer from 1 to 5, preferably from 1 to 3, more preferably 1 or 2;
x is an integer from 1 to 5, preferably from 1 to 3, more preferably 1 or 2;
alkyl is a straight or branched alkyl residue having 1-10 carbon atoms, preferably 1-3 carbon atoms; and if desired, a pharmaceutically acceptable carrier.

14. An aminocampthotecin prodrug ester having formula 20 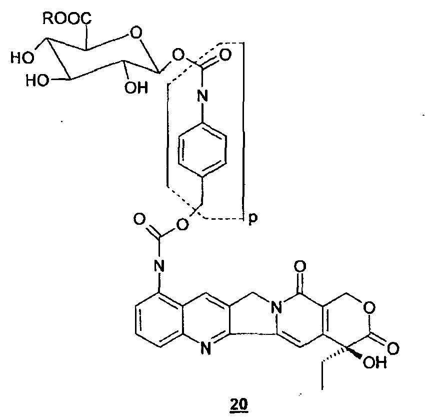 wherein:
R = CH₃; CH₂=CH-CH₂ ; CH=C-(CH₂)ₓ- ; HO-CH₂-CH₂- ; CH₃O-CH₂-CH₂- ; or a residue having formula 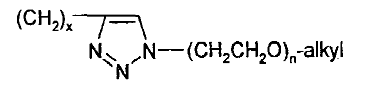
n is an integer from 1 to 40, preferably from 1 to 10, more preferably from 1 to 6;
p is an integer from 1 to 5, preferably from 1 to 3, more preferably 1 or 2;
x is an integer from 1 to 5, preferably from 1 to 3, more preferably 1 or 2;
alkyl is a straight or branched alkyl residue having 1-10 carbon atoms, preferably 1-3 carbon atoms.

15. A process for the preparation of an aminocampthotecin prodrug ester having formula 20 as defined in claim 14, wherein R is Me, allyl or (CH₂)ₓ-C≡CH, comprising the reaction of a compound of formula 11a, 11b, 11c or 16a, 16b and 16c: 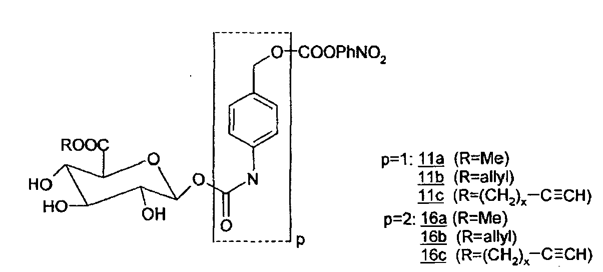 obtained according to a process according to claim 2, with a 9-aminocampthotecin of formula 19 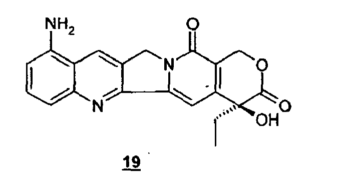 to obtain an aminocampthotecin prodrug ester of formula 20a or 20b 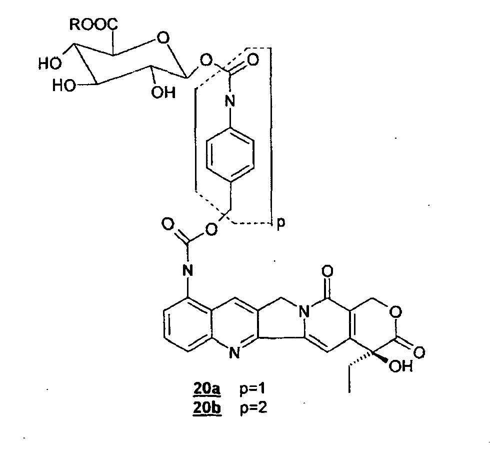 wherein: R is Me, allyl or (CH₂)ₓ-C≡CH, and p is 1 or 2.

16. An antitumor composition for administration orally, topically or by injection, containing as active ingredient an aminocamthotecin prodrug ester of formula 20a or 20b wherein R, n, x, p and alkyl are defined as in claim 14, and, if desired, a pharmaceutically acceptable carrier.
